# EUROPEAN PATENT APPLICATION

(11) **EP 3 677 631 A1**
(43) Date of publication of application: **08.07.2020**
(21) Application number: 19822679.7
(22) Date of filing: 16.04.2019
(51) Int. Cl.: C08L 1/02, C08K 7/26

(54) **MOLECULAR SIEVE/FIBER COMPOSITE MATERIAL AND PREPARATION METHOD THEREFOR**

(30) Priority: 18.06.2018 CN 201810625864
(71) Applicant: Zhejiang University, Hangzhou, Zhejiang 310058 (CN)
(72) Inventor: FAN, Jie, Hangzhou, Zhejiang 310058 (CN); YU, Lisha, Hangzhou, Zhejiang 310058 (CN); XIAO, Liping, Hangzhou, Zhejiang 310058 (CN); CHEN, Hao, Hangzhou, Zhejiang 310058 (CN); SHANG, Xiaoqiang, Hangzhou, Zhejiang 310058 (CN)
(74) Representative: Hanna Moore + Curley
(86) International application number: PCT/CN2019/082930
(87) International publication number: WO 2019/242390

(57) **Abstract**

The disclosure provides a molecular sieve/fiber composite material comprising molecular sieves and a fiber, the molecular sieves are distributed on the fiber surface and directly contact the fiber surface; the particle diameter D90 of the molecular sieves is 0.01 to 50 µm, the particle size D50 of the molecular sieves is 0.005 to 30 µm; the molecular sieves are distributed uniformly on the fiber surface. The disclosure also provides a preparation method for the molecular sieve/fiber composite material and various applications. The disclosure solves the problem of aggregation of molecular sieves on the fiber surface in molecular sieve/fiber composite material for the first time, and prepares a novel molecular sieve/fiber composite material with high strength, elastic recovery ability, and dimensional stability, making the composite material strong and durable. The molecular sieve/fiber composite material has a simple structure, low cost, strong stability, high repeatability of performance, and high practical efficiency, and provides the application in the fields of hemostasis, beauty, deodorization, sterilization, water purification, air purification, and radiation resistance.

## Description

### Technical field

The disclosure relates to the field of composite materials, in particular to a molecular sieve/fiber composite material and a preparation method thereof.

### Background of the disclosure

A substance with a regular microporous channel structure and a function of screening molecules is called molecular sieve. TO₄ tetrahedron (T is selected from Si, Al, P, B, Ga, Ge, etc.) is the most basic structural unit (SiO₄, AlO₄, PO₄, BO₄, GaO₄, GeO₄, etc.) constituting the molecular sieve skeleton, which is bound by a common oxygen atom to form a three-dimensional network structure. This combination forms holes and pores with a molecular level and a uniform pore size. Skeleton T atoms usually refer to Si, Al or P atoms, and in a few cases to other atoms, such as B, Ga, Ge, etc. For example, a zeolite is an aluminosilicate molecular sieve, which is an aluminosilicate with the ability of sieving molecules, adsorption, ion exchange, and catalysis. The general chemical composition of zeolite is: (M)_{2/n}O·xAl₂O₃·ySiO₂·pH₂O, M stands for metal ion (such as K⁺, Na⁺, Ca²⁺, Ba²⁺, etc.), n stands for valence of metal ion, and x stands for mole of Al₂O₃ , Y represents the mole number of SiO₂, and p represents the mole number of H₂O. The molecular sieve may be X-type molecular sieve, Y-type molecular sieve, A-type molecular sieve, ZSM-5 molecular sieve, chabazite, beta molecular sieve, mordenite, L-type molecular sieve, P-type molecular sieve, merlinoite, AlPO4-5 molecular sieve, AlPO4-11 molecular sieve, SAPO-31 molecular sieve, SAPO-34 molecular sieve, SAPO-11 molecular sieve, BAC-1 molecular sieve, BAC-3 molecular sieve, or BAC-10 molecular sieve. The wide application of molecular sieve material (such as adsorption and separation, ion exchange, catalysis) is inseparable from its structural characteristics. For example, the performance of adsorption and separation depends on the size and volume of the pores of the molecular sieve; the performance of ion exchange depends on the number and position of the cations in the molecular sieve and the permeability of the pores; the shape selectivity shown in the catalytic process is related to the size and direction of the pores of the molecular sieve; the intermediate product and the final product in the catalytic reaction are related to the channel dimension of the molecular sieve or its cage structure.

Fiber refers to a soft slender body with a length many times larger than its diameter, a diameter of several micrometers to hundreds of micrometers, and a slender substance with considerable flexibility and strength. The fibers in the present disclosure refers to organic fibers. The organic fibers can be divided into two categories: one is natural fiber, such as cotton, wool, silk, and hemp; the other is chemical fiber, which is made by chemical processing of natural or synthetic polymer compounds. According to the source and chemical structure of the polymer, chemical fiber can be divided into artificial fiber and synthetic fiber. Artificial fibers are divided into regenerated protein fibers, regenerated cellulose fibers, and cellulosic fibers. Synthetic fibers are divided into carbon chain fibers (the macromolecule main chain is composed of C-C) and heterochain fibers (the macromolecule main chain contains other elements, such as N, O, etc.). Carbon chain fibers include polyacrylonitrile fibers, polyvinyl acetal fibers, polyvinyl chloride fibers, fluorine-containing fibers and so on. Heterochain fibers include polyamide fibers, polyester fibers, polyurethane elastic fibers, polyurea fibers, polytoluene fibers, polyimide fibers, polyamide-polyhydrazine fibers, polybenzimidazole fibers, and so on.

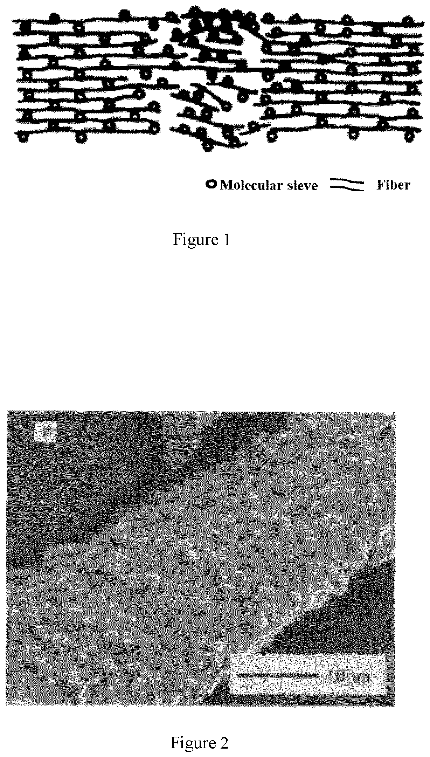

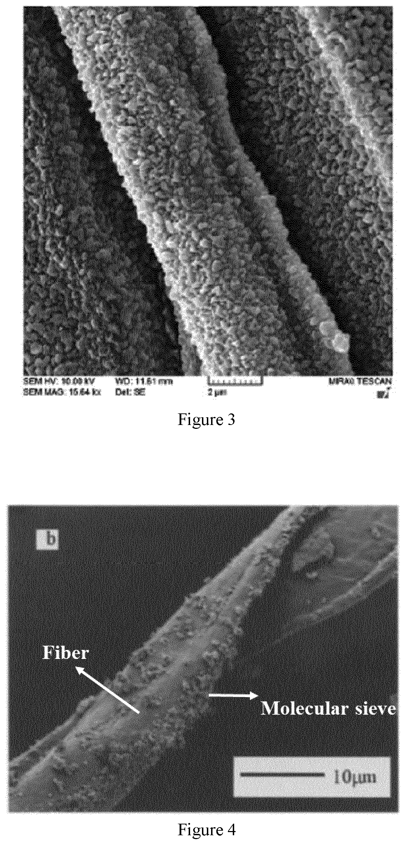

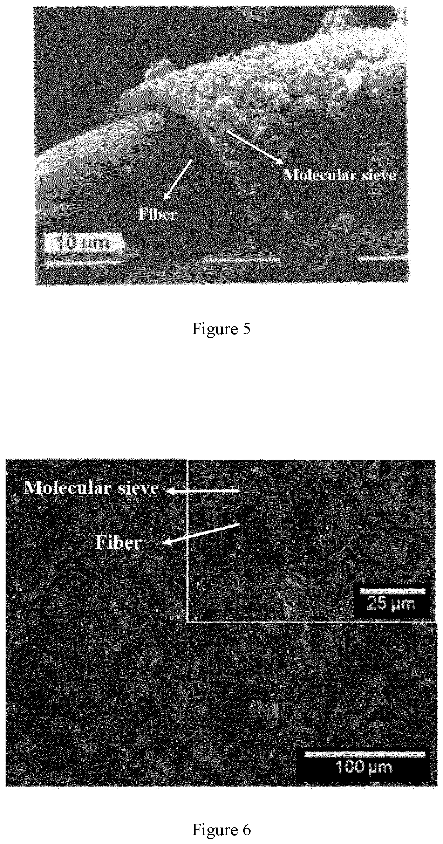

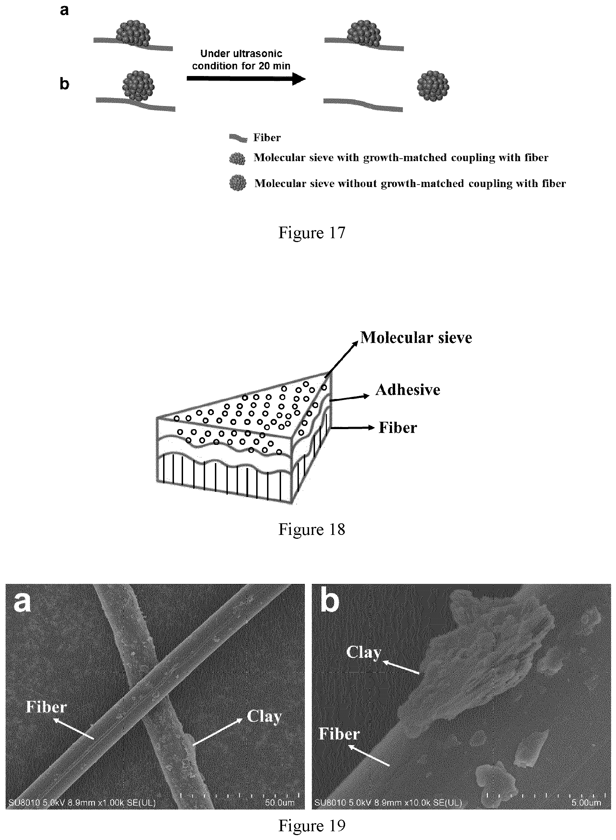

In order to facilitate the use of molecular sieves, the molecular sieves need to be supported on a suitable support. Fiber has good physical properties such as flexibility, elasticity, strength, and weavability. It is one of the ideal choices for molecular sieve carriers. Inorganic fibers are mainly glass fibers, quartz glass fibers, carbon fibers, boron fibers, ceramic fibers, metal fibers, silicon carbide fibers, and the like. The surface compounds of some inorganic fibers (such as silica fibers) can chemically react with molecular sieves, which can cause molecular sieves to bind to the surface of inorganic fibers. However, inorganic fibers are brittle, easy to break, and easy to wear, which makes them unsuitable as a flexible carrier for molecular sieves. Therefore, inorganic fibers are not within the scope of the fibers described in the present disclosure. For organic fibers, because the polar groups (such as hydroxyl group) on the fiber surface are inert and inactive, the interaction between the molecular sieve and the fiber is very weak. At present, most molecular sieves are sprayed or impregnated on the fibers. The molecular sieves and fibers are simply physically mixed, and the binding force is weak. As a result, the molecular sieve on the fiber has less adsorption and is easy to fall off. In the prior art, in order to better combine molecular sieves on the fiber surface, pretreatment of the fibers (destruction of the fiber structure), adhesive bonding, and blend spinning of molecular sieve and fiber are used:
(1) Pretreatment of the fibers. The pretreatment refers to a treatment method that destroys the fiber structure. In the prior art, in order to enable the molecular sieve to be directly bonded to the fiber, the fiber must be pretreated first. Pretreatment methods mainly include chemical treatment, mechanical treatment, ultrasonic treatment, microwave treatment, and so on. The method of chemical treatment is divided into treatment with a base compound, an acid compound, an organic solvent, etc. The base compound may be selected from any one or more of NaOH, KOH, Na₂SiO₃, etc. The acid compound may be selected from any one or more of hydrochloric acid, sulfuric acid, nitric acid, etc. The organic solvent may be selected from any one or more of ether, acetone, ethanol etc. Mechanical treatment can be by crushing or grinding fibers. The above-mentioned fiber treatment methods, although the pretreatment to a certain extent activates the polar groups (such as hydroxyl group) on the fiber surface, but seriously damages the structure of the fiber (Figure 1), destroying the fiber flexibility, elasticity and other characteristics. The fiber becomes brittle, hard and other bad phenomena, and cannot give full play to the advantages of fiber as a carrier. In addition, pretreatment of the fibers will cause molecular sieves to aggregate on the fiber surface. For example, molecular sieves are wrapped on the fiber surface with agglomerates or massive structures (Figures 2 and 3), resulting in poor fiber flexibility of fiber; or molecular sieves are partially agglomerated and unevenly distributed on the fiber surface (Figure 4); or there is a gap between the fiber and the molecular sieve (Figure 5), and the force between the molecular sieve and the fiber is weak. The aggregation of molecular sieves on the fiber surface will lead to uneven distribution of molecular sieves on the fiber surface, and cause differences in the properties across molecular sieve/fiber composite material, reducing the original specific surface area of the molecular sieve, leading to blockage of the molecular sieve channels and lowering the ability of ion exchange and pore material exchange. The pretreatment of the fiber surface is at the cost of destroying the fiber structure. Although the interaction force between the part of fiber and the molecular sieve is enhanced to form a molecular sieve/fiber composite to a certain extent, the interaction force is still not strong enough. Under external conditions, for example, a simple washing method will also cause a large number (50-60%) of molecular sieves to fall off the fiber surface (Microporous & Mesoporous Materials, 2002, 55 (1): 93-101 and US20040028900A1).
(2) Adhesive bonding. In order to increase the bonding strength between the molecular sieve and the fiber, the prior art mainly uses the adhesive to interact with the molecular sieve and the fiber to form a sandwich-like structure, and the middle layer is the adhesive, so that the molecular sieve can be indirectly bonded through the adhesive on the fiber (Figure 18). Adhesive is a substance with a stickiness that relies on a chemical reaction or physical action as a medium to connect the separated molecular sieve and the fiber material together through a binder. For adhesives, the main disadvantages include: (1) the quality of the joint cannot be inspected with the naked eye; (2) careful surface treatment of the adherend is required, such as chemical corrosion methods; (3) long curing time is needed; (4) the temperature used is too low, and the upper temperature limit for general adhesives is about 177 °C, and the upper temperature limit for special adhesives is about 371 °C; (5) most adhesives require strict process control, and especially the cleanliness of the bonding surface is higher; (6) the service life of the bonding joint depends on the environment, such as humidity, temperature, ventilation and so on. In order to ensure that the performance of the adhesive is basically unchanged within the specified period, strict attention must be paid to the method of storing the adhesive. For molecular sieves, the main disadvantages of using adhesive include: (i) uneven distribution of molecular sieves on the fiber surface; (ii) easy agglomeration of molecular sieves, reducing the effective surface area of molecular sieves, and possibly causing blockage of molecular sieve channels, and reducing ion exchange of molecular sieves, and leading to poor transfer of materials and high cost of synthetic molecular sieves. In addition, the addition of the adhesive does not significantly increase the load of the molecular sieve on the fiber; does not greatly enhance the binding of the molecular sieve to the fiber. And the molecular sieves still easily fall off the fiber surface. From a microscopic perspective, through scanning electron microscope observation, the "molecular sieve/fiber composite material" referred to in the prior art has not been observed to have a distinct bonding interface between the molecular sieve and the fiber, indicating that the bonding between the molecular sieve and the fiber through the adhesive in the prior art is not firm.

Adhesives are divided according to material source: (i) natural adhesives, including starch, protein, dextrin, animal gum, shellac, skin rubber, bone glue, natural rubber, rosin and other biological adhesives, and also include asphalt and other mineral adhesives; (ii) synthetic adhesives, mainly refers to synthetic substances, including inorganic binders such as silicate, phosphate; and epoxy resin, phenolic resin, urea resin, polyvinyl alcohol, polyurethane, polyetherimide, polyvinyl acetal, perchloroethylene resin and other resins; neoprene, nitrile rubber and other synthetic polymer compounds. According to the use characteristics: (1) water-soluble adhesives, such as starch, dextrin, polyvinyl alcohol, carboxymethyl cellulose, etc.; (2) hot-melt adhesives, such as polyurethane, polystyrene, polyacrylate, ethylene-vinyl acetate copolymer, etc.; (3) solvent-based adhesives, such as shellac, butyl rubber, etc.; (4) emulsion adhesive, mostly suspended in water, such as vinyl acetate resin, acrylic resin, chlorinated rubber, etc.; (5) solvent-free liquid adhesive, which is a viscous liquid at normal temperature, such as epoxy resin, etc. According to raw materials: (i) MS modified silane, the end of the modified silane polymer is methoxysilane; (ii) polyurethane, the full name of polyurethane is a collective name for macromolecular compounds containing repeating urethane groups on the main chain; (iii) silicones are commonly referred to as silicone oil or dimethyl silicone oil. The molecular formula is (CH₃)₃SiO(CH₃)₂SiOₙSi(CH₃)₃. It is a polymer of organic silicon oxide and a series of polydimethylsiloxanes with different molecular weight, of which viscosity increases with molecular weight.

The paper discloses an adhesive-bonded A-type molecular sieve/wool fiber composite material (Applied Surface Science, 2013, 287 (18): 467-472). In this composite, 3-mercaptopropyltrimethoxysilane is used as a binder, and the A-type molecular sieve is bonded to the surface of the wool fiber, and the content of the A-type molecular sieve on the fiber is 2.5% or less. After the silane binder was added, agglomerated molecular sieves appeared on the surface of wool fibers, which was observed through scanning electron microscopy. The active silane binder caused agglomeration of molecular sieve particles. The agglomeration of molecular sieve particles will reduce the effective surface area of the molecular sieve and the ability of material exchange.

The paper discloses an adhesive-bonded Na-LTA molecular sieve/nanocellulose fiber composite material (ACS Appl. Mater. Interfaces 2016, 8, 3032-3040). In this composite, nanocellulose fibers were immersed in a polydiallyl dimethyl ammonium chloride (polyDADMAC) aqueous solution at 60 °C for 30 minutes to achieve adsorption of LTA molecular sieves (polyDADMAC is an adhesvie). Although molecular sieves can be attached to the fiber surface by polycation adsorption of polyDADMAC, for nano Na-LTA at 150 nm, mesoporous Na-LTA and micron-sized Na-LTA on the fiber, the content is only 2.6 ± 0.6 wt% (coefficient of variation is 23.1%, calculation method is 0.6×100%/2.6 = 23.1%), 2.9 ± 0.9 wt% (coefficient of variation is 31.0%), and 12.5 ± 3.5 wt% (coefficient of variation is 28%), respectively. The molecular sieve is unevenly distributed on the fiber surface, which is difficult to achieve equal content of molecular sieve on fiber surface. Coefficient of variation is also called the "standard deviation rate", which is the ratio of the standard deviation to the mean multiplied by 100%. Coefficient of variation is an absolute value that reflects the degree of dispersion of the data. The higher the coefficient of variation, the greater the degree of dispersion of the data, indicating that the content of molecular sieves varies widely across the fiber surface. From the scanning electron microscope, it can be observed that the Na-LTA molecular sieve does not form a binding interface with the fiber (Figure 6), indicating that the bonding between the fiber and the molecular sieve is not strong, and the molecular sieve is easy to fall off the fiber surface.

The paper discloses a Y-type molecular sieve/fiber composite material bonded by an adhesive (Advanced Materials, 2010, 13 (19): 1491-1495). Although molecular sieves can be attached to the surface of plant fibers by covalent bonds of the binder, the amount of adhesion is limited (all below 5 wt%). In this composite, 3-chloropropyltrimethoxysilane was used as a binder to modify the surface of the molecular sieve to make it adhere to cotton fibers. And it fell off by 39.8% under ultrasonic conditions for 10 minutes (the retention rate of the molecular sieve on the fibers was 60.2%); it fell off by 95% under ultrasonic conditions for 60 minutes (the retention rate of the molecular sieve on the fibers was 5%), which indicates that the chemical bonding between the molecular sieve and the fiber is not strong in this technology. In order to increase the bonding strength between molecular sieve and fiber, this technology modifies polyetherimide as a binder to the fiber surface, and then attaches 3-chloropropyltrimethoxysilane-modified molecular sieve to the binder-modified fiber. Although the adhesive strength of the fiber and molecular sieve is increased to some extent under this condition, it still falls off under ultrasonic conditions. For this composite material, both the surface of the molecular sieve and the fiber interact with the binder, and the sandwich-like material is formed by the adhesive of the intermediate layer, which increases the cost of the synthesis process and reduces the effective surface area of the molecular sieve.

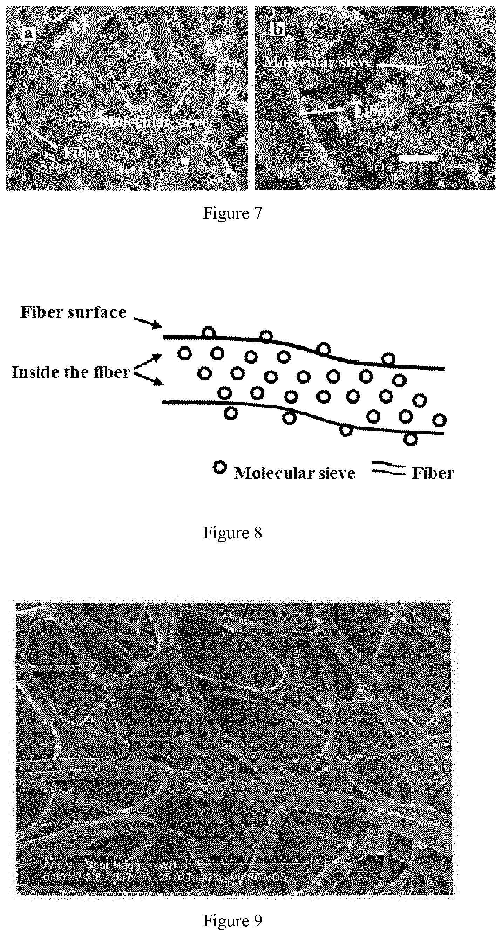

The paper discloses a NaY-type molecular sieve/fiber composite material bonded by a cationic and anionic polymer binder (Microporous & Mesoporous Materials, 2011, 145 (1-3): 51-58). NaY molecular sieves are added to cellulose and polyvinyl alcohol amine solution, and then polyacrylamide polymer solution is added to form corresponding composite materials. Scanning electron microscopy can observe that there is no bond between NaY molecular sieve and fiber, and there is no force between them. It is just a simple physical combination of the two materials so that the molecular sieve can easily fall off the fiber (Figure 7).

(3) Blend spinning of molecular sieve and fiber. The solution of molecular sieve and the solution of synthetic fiber are mixed uniformly for spinning, and the molecular sieve and fiber are simply physically combined. Molecular sieves are mostly present in the fibers and are not bound to the fiber surface (Figure 8).

US patent US7390452B2 discloses an electrospun mesoporous molecular sieve/fiber composite material. Polyetherimide (PEI) methanol solution and mesoporous molecular sieve solution were electrospun to form a composite. No molecular sieve was apparently observed on the fiber surface from the scanning electron microscope (Figure 9), which suggests most of the molecular sieve was present inside the fiber, and the internal molecular sieve was unable to exert its performance.

Chinese patent CN1779004A discloses an antibacterial viscose fiber and its preparation method. The disclosure is prepared by blending and spinning a silver molecular sieve antibacterial agent and a cellulose sulfonate solution, and the silver molecular sieve antibacterial agent accounts for 0.5 to 5% of cellulose. The silver molecular sieve is dispersed in a cellulose sulfonate solution through a 0.01% MET dispersant, and spin-molded at a bath temperature of 49 °C. Most of the molecular sieve exists in the fiber, which easily blocks the molecular sieve channels, resulting in a small effective surface area.

In summary, there are four structural forms of molecular sieve/fiber composite material made in the prior art: (1) physical mixing of molecular sieves and fibers; (2) aggregation of molecular sieves formed on the fiber surface; (3) molecular sieve and the fiber form a sandwich-like structure through an adhesive; (4) most of the molecular sieves are present inside the fiber and are not bound to the fiber surface. The four structural forms of the composite material have poor dispersibility of the molecular sieve and a small effective specific surface area, so they cannot exert their performance well. Among the first three structural forms of the composite materials, the molecular sieves have a weak binding strength with the fiber, and the molecular sieves may easily fall off the fiber surface. The effective specific surface area of the molecular sieves in the molecular sieve/fiber composite material is smaller than the original effective specific surface area of the molecular sieves. Without the addition of an adhesive, the prior art cannot achieve uniform distribution of molecular sieves that directly contact the fiber surface, cannot achieve a strong binding between the molecular sieves and the fibers of molecular sieve/fiber composite material, and cannot not remain the original large effective specific area and strong pore material exchange capacity of molecular sieves on the fibers.

According to the rules of the International Union of Pure and Applied Chemistry: substances with a pore size of less than 2 nm are called microporous materials, substances with a pore size ranging from 2 to 50 nm are called mesoporous materials, and substances with a pore size greater than 50 nm are called macroporous materials. Due to their small pores (usually 0.4 to 1.2 nm), the molecular sieves severely affect the transport of substances for their internal active sites, which extremely limits their performance as catalysts for macromolecular reactions (catalytic cracking, etc.). In order to solve this problem, people have prepared mesoporous molecular sieves with the advantages of microporous and mesoporous materials. The advantages of applying mesoporous molecular sieves to catalytic materials are: (1) the reaction rate is significantly improved for catalytic reactions controlled by diffusion; (2) the selectivity of certain products can be improved due to the increase in mass transfer rate; (3) the reactive molecules are more accessible to the active sites; (4) the catalyst is not easily deactivated and has a longer life; (5) the catalyst can be uniformly dispersed in the secondary pores when the molecular sieve as a carrier.

There are two methods for synthesizing mesoporous molecular sieves in the prior art:
(1) Post-treatment method of microporous molecular sieve (e.g. high-temperature heat treatment, high-temperature water vapor treatment, acid treatment, alkali treatment of microporous molecular sieve, etc.). Through the treatment, aluminum or silicon can be selectively removed from the framework, and secondary pores are generated in the formed molecular sieves. However, the framework of molecular sieve is easy to collapse during post-processing of dealumination or desiliconization. In addition, the connectivity between mesoporous channels is not good, and the diffusion and transmission of macromolecules is not improved. Besides, a large amount of waste liquid is generated during the synthesis process, which not only pollutes the environment but also wastes reagents, so this method is greatly limited in practical applications.
(2) Template method (soft template method and hard template method).

If the post-treatment method of microporous molecular sieve is not used, only the template method can be used, and a templating agent is necessarily used. The template method is divided into soft template method and hard template method. The templating agent of the soft template method mainly includes a polymer, a surfactant, an organosilane, and so on. Xiao Fengshou's research team successfully synthesized a mesoporous β molecular sieve for the first time using a polymer (polydiallyldimethylammonium chloride) as a templating agent in 2006. The results showed that the molecular sieve crystal contains both micropores of about 0.8 nm and mesopores of 5-20 nm (Angew. Chem., 2006, 118 (9): 3162.). Subsequently, this method was extended to the synthesis of mesoporous ZSM-5, X-type and Y-type molecular sieves. Gu et al. added cetyltrimethylammonium bromide (CTABr) as a mesoporous template during the synthesis of molecular sieves, and added co-solvent tert-butanol (TBA) and pore expander mesitylene (TMB) simultaneously, to synthesize Y-type molecular sieves with mesoporous structure (Chem. Mater., 2010, 22: 2442.). Although the soft template method can synthesize mesoporous molecular sieves, the soft template is generally expensive and requires strict experimental conditions. Therefore, this method generally cannot meet the requirements of industrial production.

In the process of synthesizing molecular sieves, the hard templating agents do not interact with the silicon or aluminum source in the hard template method, but only serve the purpose of occupying space. After the crystallization reaction is completed, the templating agents are removed by means such as calcining, to obtain mesoporous molecular sieves. Templating agents that can be used as the hard template method include nano-CaCO₃, nano-MgO, corn starch, carbon materials (e.g. ordered mesoporous carbon), and so on. The templating agents that can be used in the hard template method have large limitations in terms of type or shape. Especially after the hard templating agent is removed, the connectivity of the obtained mesoporous channels is relatively poor, which limits practical application of molecular sieves.

In summary, the methods of synthesizing mesoporous molecular sieves still have disadvantages in the prior art: (1) The mesoporous molecular sieves synthesized by the template method (including the hard template method and the soft template method) will affect the pore channel connectivity of molecular sieves and limit the catalysis on the surface of molecular sieves and other applications in the presence of the templating agents. (2) Mesoporous molecular sieves prepared by the hard template method generally have poor connectivity of the mesoporous structure, while the soft template method is relatively expensive, which limits the application of the template method to a certain extent. (3) The post-treatment method of microporous molecular sieves can not only form secondary pores in the molecular sieves, but also change the silicon-aluminum ratio of the molecular sieve framework, thereby adjusting the acidity of the molecular sieves. However, this method is easy to cause the collapse of molecular sieve framework, poor controllability of secondary pores, and environmental pollution.

Therefore, the synthesis of mesoporous molecular sieves in the prior art is complicated and time-consuming, and the molecular sieves cannot achieve the expected catalytic performance, and organic templating agents have great environmental pollution. In the prior art, there is no synthesis method of mesoporous molecular sieves, which does not require a templating agent, and has low cost, simple and environmentally friendly process, and meets industrial production.

Humans (including animals) can be injured in a variety of situations. In some cases, the trauma and bleeding are minor, and in addition to the application of simple first aid, only regular coagulation is required to stop bleeding normally. Unfortunately, however, massive bleeding can occur in other settings. These situations often require specialized equipment and materials, and professionally trained personnel to implement appropriate assistance. If such assistance is not readily available, excessive blood loss may result in death. Bleeding is a very serious global problem. It is estimated that 1.9 million people worldwide die from bleeding every year, of which 1.5 million die from physical trauma. Among them, the deaths of these 1.5 million people have caused nearly 75 million years of life loss worldwide, which is about half of the life loss caused by cancer (170 million years). Therefore, in the emergency treatment of sudden accidents in daily life, the hemostasis during the operation of the hospital to the patient, especially the rescue of the wounded soldiers during the war, the effective rapid hemostasis for the patient is very important.

Molecular sieves have good hemostatic effects, are suitable for emergency hemostasis, especially aortic bleeding, and are cheap, stable, and easy to carry. Molecular sieves rely on van der Waals forces during the adsorption process. The distance between molecules during the mutual attraction process is reduced, and the potential energy of the molecules is converted to internal energy to release energy. Therefore, the molecular sieves absorb water and release heat. In the prior art, molecular sieves mainly cover wounds in the form of granules or powder to stop bleeding, and the wounded may feel uncomfortable. Because the hemostatic materials need to completely cover the wound, powder and granular molecular sieves are used as hemostatic materials in very large amounts, which causes the molecular sieves to absorb a large amount of water and exotherm on the wound and easily burn the wound. During use, the molecular sieves will stick to the wound, which is very difficult to clean. It needs multiple flushes to remove them, which easily leads to secondary bleeding. The granular molecular sieves need to be adhered by an adhesive so that the molecular sieves cannot contact the blood of the wound sufficiently, which affects the hemostatic effect of molecular sieve. At the same time, during the use of powder and granular molecular sieves, due to direct contact with the wound, the molecular sieves adhered on the wound have the risk of entering blood vessels or other tissues, and they are easy to remain in the lumen of the blood vessel to form a thrombus to block peripheral artery flow.

Dispersing molecular sieves (or inorganic hemostatic materials) on the fibers in a small size can solve the problem of molecular sieve materials sticking to the wound and the difficulty of cleaning up the wound to a certain extent, reduce the amount of molecular sieves, and dilute the exothermic effect caused by water absorption. However, when the existing molecular sieve/fiber composite materials are used as hemostatic fabric materials, the molecular sieve/fiber composite materials prepared in the prior art have four structural forms: (1) physical mixing of molecular sieves and fibers; (2) aggregation of molecular sieves formed on the fiber surface; (3) molecular sieve and the fiber form a sandwich-like structure through an adhesive; (4) most of the molecular sieves are present inside the fiber and are not bound to the fiber surface. The composite materials of these four structural forms have poor dispersibility and small effective specific surface area of the molecular sieves, insufficient contact with blood during use, and poor hemostatic effect. The effective specific surface area of the molecular sieves in the molecular sieve/fiber composite material is smaller than the original effective specific surface area of the molecular sieves. Among the first three types of composite materials, molecular sieves easily fall off the fiber surface. Some detached molecular sieves will stick to the wound and some will enter the blood circulation. Molecular sieves tend to remain in blood vessels, inducing the risk of thrombosis. On the other hand, the properties of silicate inorganic hemostatic materials (e.g. clay) are similar to molecular sieves. The composite materials formed by inorganic hemostatic materials and fibers also have the above-mentioned four defective structural forms, which seriously affect the performance and safety of hemostatic materials.

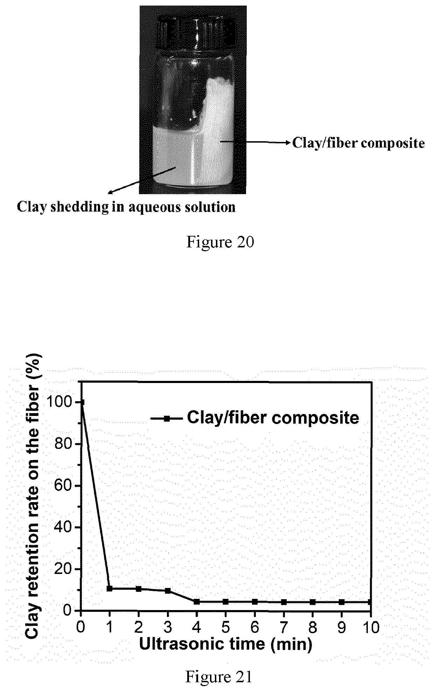

Z-Medica Co., Ltd. developed Combat Gauze, a hemostatic product known as a "life-saving artifact". This product is an emergency hemostatic product recommended by Committee on Tactical Combat Casualty Care (Co-TCCC) for the US military. At present, it is used as an important first-aid device for military equipment and ambulances. US patent US8114433B2, Chinese patent CN101541274B, and CN101687056B disclose that the technology of this type of hemostatic product is a device capable of providing hemostatic effect on bleeding wounds, using an adhesive to attach a clay material to the surface of gauze. However, the adhesive not only reduces the contact area of the clay material with the blood, but also has a weak binding strength between the clay material and the gauze fibers. After the hemostatic material product made according to this technology encounters water, the clay material on the gauze surface is still very easy to fall off the gauze fiber (Figure 20). Clay retention rate on the gauze fiber is 10% or less under ultrasonic condition for 1 minute; clay retention rate on the gauze fiber is 5% or less under ultrasonic condition for 5 minutes (Figure 21); clay retention rate on the gauze fiber is 5% or less under ultrasonic condition for 20 minutes. This defective structural form limits the hemostatic properties of the hemostatic product and risks causing sequelae or other side effects (such as thrombus).

Chinese patent CN104888267A discloses a medical hemostatic spandex fiber and a preparation method thereof. The method for preparing medical hemostatic spandex fiber includes the following steps: (1) preparing a polyurethane urea stock solution; (2) grinding the inorganic hemostatic powder and dispersant in a dimethylacetamide solvent to obtain a hemostatic solution; (3) placing the polyurethane urea stock solution and hemostatic solution in a reaction container, and weaving them into a spandex fiber through a dry spinning process. The inorganic hemostatic powder is one or more of diatomite, montmorillonite, zeolite, bioglass and halloysite nanotubes. A large part of the inorganic hemostatic powder in the hemostatic spandex fiber is inside the fiber, which cannot fully contact with the blood and cannot exert its hemostatic effect. Although the amount of inorganic hemostatic powder is increased, the hemostatic effect is not good.

The problems with hemostatic materials in the prior art are: (1) powder and granular molecular sieves (or inorganic hemostatic materials) absorb a large amount of water and release heat on the wound; (2) the composite materials formed from molecular sieves (or inorganic hemostatic materials) and fiber have poor dispersibility and small effective specific surface area of molecular sieves, and represent insufficient contact with blood during use and poor hemostatic performance; (3) the binding strength between molecular sieve (or inorganic hemostatic material) and fiber is weak, and molecular sieves are easy to fall off from the fiber surface during practical use. The retention rate of molecular sieves is low, and the hemostatic material easily loses the hemostatic effect. Without the addition of an adhesive, the prior art cannot achieve a strong binding between the molecular sieve (or inorganic hemostatic material) and the fiber, cannot prevent molecular sieve fall off, cannot achieve good dispersibility of molecular sieve on the fiber surface, and cannot remain effective specific surface area of molecular sieve and cannot possess excellent hemostatic function of molecular sieve and fiber composite.

### Summary of the disclosure

In view of the shortcomings of the prior art, the first technical problem to be solved by the present disclosure is to solve the problem of molecular sieve aggregation on the fiber surface in a molecular sieve/fiber composite material, and to provide a molecular sieve/fiber composite material with strong binding between the molecular sieve and the fiber, uniform distribution of molecular sieve on the fiber surface without adding an adhesive, and the molecular sieve in the composite material maintains the original large effective specific surface area and strong substance exchange capacity of pore.

The present disclosure adopts the following technical solutions:
The present disclosure unexpectedly obtained a novel material by a simple method, a molecular sieve/fiber composite material, the molecular sieves are distributed on the fiber surface and directly contact the fiber surface, and the particle diameter D90 of the molecular sieves is 0.01 to 50 µm, the particle size D50 of the molecular sieves is 0.005 to 25 µm, and the molecular sieves are distributed uniformly on the fiber surface of the fiber.

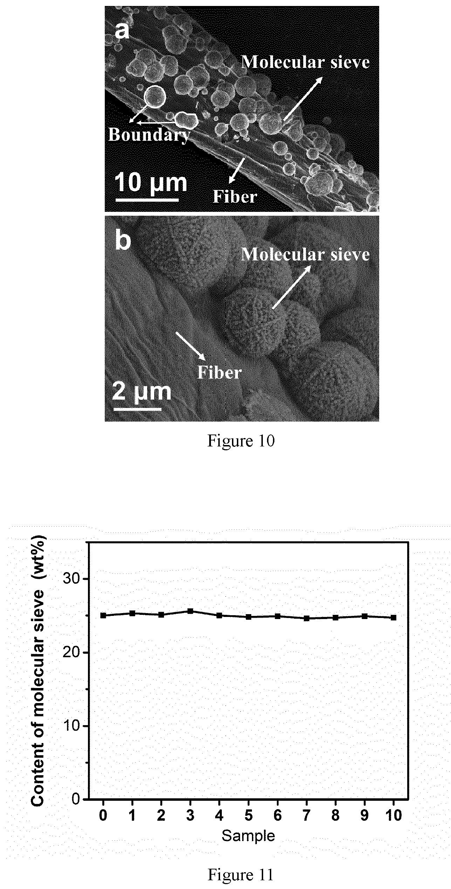

D50 refers to the particle size corresponding to the cumulative particle size distribution percentage of the molecular sieve microparticles on the surface of the molecular sieve/fiber composite material reaching 50%. Its physical meaning is that molecular sieve microparticles with a particle size larger than it account for 50%, and molecular sieve microparticles with a particle size smaller than it also account for 50%. D50 is also called median particle size, which can represent the average particle size of molecular sieve microparticles. The molecular sieve microparticle is the molecular sieve geometry with a certain shape and a size smaller than 50 µm, which retains the boundary (Figure 10a) of growth shape of the original molecular sieve.

D90 refers to the particle size corresponding to the cumulative particle size distribution percentage of the molecular sieve microparticles on the surface of the molecular sieve/fiber composite material reaching 90%. Its physical meaning is that the molecular sieve microparticles with a particle size larger than it account for 10%, and the molecular sieve microparticles with a particle size smaller than it account for 90%.

The detection method of "uniform distribution of molecular sieves on the fiber surface" is: randomly taking n samples of the molecular sieve/fiber composite material at different locations and analyzing the content of the molecular sieve on the fiber surface, where n is a positive integer greater than or equal to 8. The coefficient of variation is also called the "standard deviation rate", which is the ratio of the standard deviation to the mean multiplied by 100%. The coefficient of variation is an absolute value that reflects the degree of dispersion of the data. The smaller the value of the coefficient of variation, the smaller the degree of dispersion of the data, indicating that the smaller the difference in the content of molecular sieves on the fiber surface, the more uniform the distribution of molecular sieves on the fiber surface. The coefficient of variation of the content of the molecular sieves in the n samples is ≤ 15%; preferably, the coefficient of variation of the content of the molecular sieves is ≤ 10%; preferably, the coefficient of variation of the content of the molecular sieves is ≤ 5%; preferably, the coefficient of variation of the content of the molecular sieves is ≤ 2%; preferably, the coefficient of variation of the content of the molecular sieves is ≤ 1%; preferably, the coefficient of variation of the content of the molecular sieves is ≤ 0.5%; preferably, the coefficient of variation of the content of the molecular sieves is ≤ 0.2%.

The coefficient of variation of the content of the molecular sieves in the n samples according to the present disclosure is ≤ 15%, which is defined as the uniform distribution of molecular sieves on the fiber surface. Preferably, the coefficient of variation of the content of the molecular sieves in the n samples according to the present disclosure is ≤ 10%, which is defined as the uniform distribution of molecular sieves on the fiber surface. Preferably, the coefficient of variation of the content of the molecular sieves in the n samples according to the present disclosure is ≤ 5%, which is defined as the uniform distribution of molecular sieves on the fiber surface. Preferably, the coefficient of variation of the content of the molecular sieves in the n samples according to the present disclosure is ≤ 2%, which is defined as the uniform distribution of molecular sieves on the fiber surface. Preferably, the coefficient of variation of the content of the molecular sieves in the n samples according to the present disclosure is ≤ 1%, which is defined as the uniform distribution of molecular sieves on the fiber surface. Preferably, the coefficient of variation of the content of the molecular sieves in the n samples according to the present disclosure is ≤ 0.5%, which is defined as the uniform distribution of molecular sieves on the fiber surface. Preferably, the coefficient of variation of the content of the molecular sieves in the n samples according to the present disclosure is ≤ 0.2%, which is defined as the uniform distribution of molecular sieves on the fiber surface.

For example, taking 10 samples of molecular sieve/fiber composite materials at different locations, the average content of molecular sieves on the fibers in the 10 samples is 25 wt%, the standard deviation of the samples is 0.1 wt%, and the coefficient of variation is 0.4%, indicating that the molecular sieves are uniformly distributed on the fiber surface (Figure 11).

In some embodiments, the particle diameter D90 of the molecular sieve is 0.1 to 30 µm, and the particle diameter D50 of the molecular sieve is 0.05 to 15 µm; preferably, the particle diameter D90 of the molecular sieve is 0.5 to 20 µm, and the particle diameter D50 of the molecular sieve is 0.25 to 10 µm; preferably, the particle diameter D90 of the molecular sieve is 1 to 15 µm, and the particle diameter D50 of the molecular sieve is 0.5 to 8 µm; more preferably, the particle diameter D90 of the molecular sieve is 5 to 10 µm, and the particle diameter D50 of the molecular sieve is 2.5 to 5 µm.

In some embodiments, the adhesive content of the contact surface between the molecular sieve and the fiber is zero.

In some embodiments, the surface of molecular sieve contacted with the fiber is defined as inner surface, and the inner surface is a rough planar surface matched with the fiber surface; and growth-matched coupling is formed between the molecular sieve and the fiber on the inner surface of the molecular sieve, so that the molecular sieve has a strong binding strength with the fiber. The surface of molecular sieve uncontacted with the fiber is defined as outer surface, and the outer surface is a non-planar surface.

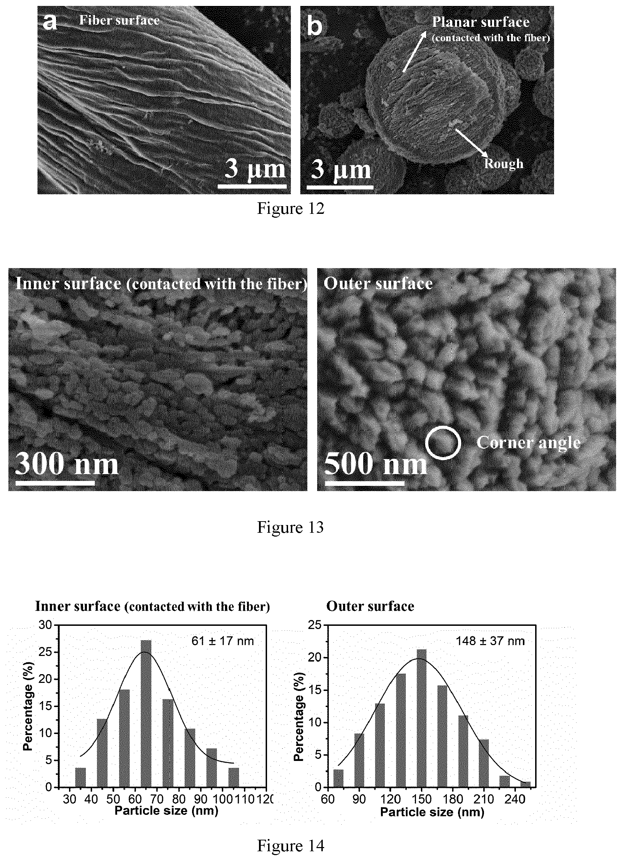

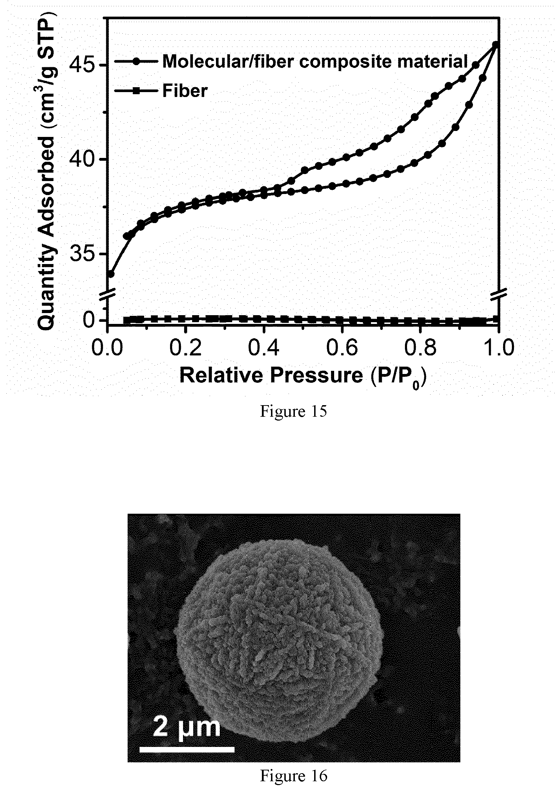

There is a complete and uniform growth surface around the molecular sieve microparticles from traditional solution growth method of molecular sieve microparticles (Figure 16). Different from the traditional solution growth method of molecular sieve microparticles, the growth-matched coupling is that the molecular sieve microparticles cooperate with the fiber surface to grow a tightly-coupled coupling interface with the fiber as shown in Figure 12, so that the molecular sieve has a strong binding strength with the fiber.

The detection method for forming the growth-matched coupling is: for molecular sieve/fiber composite material, the retention rate of the molecular sieve on the fiber is greater than or equal to 90% under ultrasonic condition for 20 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, the detection method for forming the growth-matched coupling is: for molecular sieve/fiber composite material, the retention rate of the molecular sieve on the fiber is greater than or equal to 90% under ultrasonic condition for 40 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, the detection method for forming the growth-matched coupling is: for molecular sieve/fiber composite material, the retention rate of the molecular sieve on the fiber is greater than or equal to 90% under ultrasonic condition for 60 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, both the inner surface and outer surface are composed of molecular sieve nanoparticles.

The molecular sieve nanoparticles are particles formed by molecular sieve growing in a nanometer-scale size (2 to 500 nm).

In some embodiments, the average size of the molecular sieve nanoparticles of outer surface is larger than the average size of the molecular sieve nanoparticles of inner surface.

In some embodiments, the molecular sieve is independently dispersed on the fiber surface, that is, the molecular sieves do not aggregate on the fiber surface. And the molecular sieves are independently dispersed on the fiber surface so that the fiber maintains the original physical properties of flexibility and elasticity. The independent dispersion means that each molecular sieve microparticle has its own independent boundary; as shown in Figure 10a, the boundary of each molecular sieve microparticle is clearly visible.

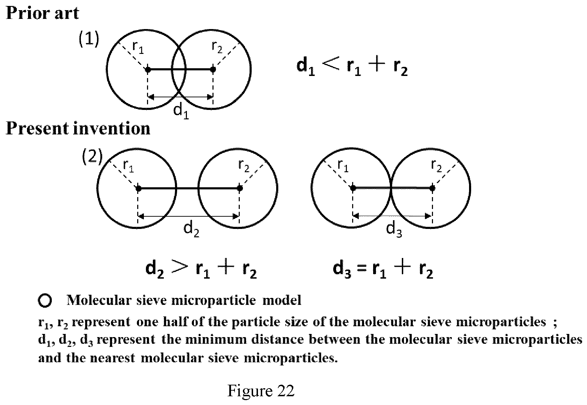

As an example, the aggregation of molecular sieves on the fiber surface (e.g. molecular sieves are distributed on the fiber surface in an agglomerated or lumpy structure) refer to the partial or full overlap of the molecular sieve microparticles and their nearest neighbor molecular sieve microparticles in space; that is, the minimum distance between the molecular sieve microparticles is less than one half of the sum of the particle size of the two molecular sieve microparticles (as shown in Figure 22(1), d < r₁+r₂).

Different from the aggregation of molecular sieve on the fiber surface, the independent dispersion means that the molecular sieve microparticles are dispersed on the fiber surface with a gap between each other, and the independent dispersion means the minimum distance between the molecular sieve microparticle and the nearest molecular sieve microparticle is greater than or equal to one half of the sum of the particle sizes of the two molecular sieve microparticles (as shown in Figure 22(2), d≥r₁+r₂), which indicates the boundary between the adjacent molecular sieve microparticles.

Because the molecular sieves are independently dispersed on the fiber surface, they are not distributed on the fiber surface in an agglomerated or lumpy structure, which promotes the uniform distribution of the molecular sieves on the fiber surface.

In some embodiments, all the molecular sieves in the molecular sieve/fiber composite material are distributed on the fiber surface.

In some embodiments, the molecular sieve nanoparticles of outer surface are particles with corner angles.

In some embodiments, the molecular sieve nanoparticles of inner surface are particles without corner angles. The nanoparticles without corner angles make the inner surface of the molecular sieve match the fiber surface better, which is beneficial to the combination of the molecular sieve and the fiber.

In some embodiments, the average size of the molecular sieve nanoparticles of inner surface is 2 to 100 nm; preferably, the average size of the molecular sieve nanoparticles of inner surface is 10 to 60 nm.

In some embodiments, the average size of the molecular sieve nanoparticles of outer surface is 50 to 500 nm; preferably, the average size of the molecular sieve nanoparticles of outer surface is 100 to 300 nm.

In some embodiments, the non-planar surface is composed of any one or combination of non-planar curves or non-planar lines. For example, the non-planar surface is composed of non-planar curves. Preferably, the non-planar surface is a spherical surface, and the spherical surface increases the effective area of contact with a substance. The spherical surface is made up of non-planar curves. In another example, the non-planar surface may be composed of non-planar curves and non-planar lines. In some embodiments, the non-planar line may be a polygonal line.

In some embodiments, the molecular sieve is a mesoporous molecular sieve.

In some embodiments, the content of the molecular sieve accounts for 0.05 to 80 wt% of the molecular sieve/fiber composite material; preferably, the content of the molecular sieve accounts for 1 to 50 wt% of the molecular sieve/fiber composite material; preferably, the content of the molecular sieve accounts for 5 to 35 wt% of the molecular sieve/fiber composite material; preferably, the content of the molecular sieve accounts for 10 to 25 wt% of the molecular sieve/fiber composite material; more preferably, the content of the molecular sieve accounts for 15 to 20 wt% of the molecular sieve/fiber composite material.

In some embodiments, the molecular sieve may be selected from the group consisting of aluminosilicate molecular sieve, phosphate molecular sieve, borate molecular sieve, heteroatom molecular sieve, and combination thereof.

Further, the aluminosilicate molecular sieve can be selected from the group consisting of X-type molecular sieve, Y-type molecular sieve, A-type molecular sieve, ZSM-5 molecular sieve, chabazite, β-molecular sieve, mordenite, L-type molecular sieve, P-type molecular sieve, merlinoite, and combination thereof.

Further, the phosphate molecular sieve can be selected from the group consisting of AlPO₄-5 molecular sieve, AlPO₄-11 molecular sieve, SAPO-31 molecular sieve, SAPO-34 molecular sieve, SAPO-11, and combination thereof.

Further, the borate molecular sieve can be selected from the group consisting of BAC-1 molecular sieve, BAC-3 molecular sieve, BAC-10 molecular sieve, and combination thereof.

Further, the heteroatom molecular sieve is that a heteroatom element replaces part of silicon, aluminum, and phosphorus of the molecular sieve framework to form a molecular sieve containing other elements.

Further, the heteroatom element can be selected from the group consisting of transition metal elements in the fourth, fifth, sixth periods, and combination thereof.

In some embodiments, the molecular sieve is a molecular sieve after metal ion exchange. The metal ion is selected from the group consisting of strontium ion, calcium ion, magnesium ion, silver ion, zinc ion, barium ion, potassium ion, ammonium ion, copper ion, and combination thereof.

In some embodiments, the fiber is a polymer containing hydroxyl groups in a repeating unit.

Further, the fiber is selected from the group consisting of silk fiber, chitin fiber, rayon fiber, acetate fiber, carboxymethyl cellulose, bamboo fiber, cotton fiber, linen fiber, wool, wood fiber, lactide polymer fiber, glycolide polymer fiber, polyester fiber (abbreviated as PET), polyamide fiber (abbreviated as PA), polypropylene fiber (abbreviated as PP), polyethylene fiber (abbreviated as PE), polyvinyl chloride fiber (abbreviated as PVC), polyacrylonitrile fiber (abbreviated as acrylic fiber, artificial wool), viscose fiber, and combination thereof.

Further, the polyester fiber refers to a polyester obtained by polycondensation of a monomer having both a hydroxyl group and a carboxyl group, or a polyester obtained by polycondensation of an aliphatic dibasic acid and an aliphatic diol, or polyester or copolyester made from aliphatic lactone through ring-opening polymerization, and the molecular weight of the aliphatic polyester is 50,000 to 250,000. The polyester obtained by polycondensation of a monomer having both a hydroxyl group and a carboxyl group is a polylactic acid obtained by direct polycondensation of lactic acid; the polyester obtained by polycondensation of an aliphatic dibasic acid and an aliphatic diol is polybutylene succinate, polyhexanediol sebacate, polyethylene glycol succinate or polyhexyl succinate; polyester produced from ring-opening polymerization of aliphatic lactones is polylactic acid obtained by ring-opening polymerization of lactide, and polycaprolactone obtained by ring-opening polymerization of caprolactone; copolyester is poly(D,L-lactide-co-glycolide).

Further, the polyamide fiber refers to polyhexamethylene adipamide obtained by polycondensation of diamine and diacid, and the chemical structure formula of its long chain molecule is: H-[HN(CH₂)_{X}NHCO(CH₂)_{Y}CO]n-OH; or obtained by polycondensation or ring-opening polymerization of caprolactam, the chemical structural formula of its long chain molecule is: H-[NH(CH₂)_{X}CO]ₙ-OH.

In some embodiments, the molecular sieve/fiber composite material is prepared by an in-situ growth method.

In some embodiments, the in-situ growth method includes the following steps:
(i) prepare a molecular sieve precursor solution and mix it with the fiber;
(ii) the mixture of fiber and molecular sieve precursor solution in step (i) is processed with heat treatment to obtain a molecular sieve/fiber composite material.

In some embodiments, the molecular sieve precursor solution does not include a templating agent.

In some embodiments, in the step (ii), the temperature of the heat treatment is 60 to 220 °C, and the time of heat treatment is 4 to 240 h.

In some embodiments, in the step (i), mass ratio of the fiber to the molecular sieve precursor solution is 1:0.5 to 1:1000; preferably, in the step (i), mass ratio of the fiber to the molecular sieve precursor solution is 1:0.8 to 1:100; preferably, in the step (i), mass ratio of the fiber to the molecular sieve precursor solution is 1:1 to 1:50; preferably, in the step (i), mass ratio of the fiber to the molecular sieve precursor solution is 1:1.5 to 1:20; preferably, in the step (i), mass ratio of the fiber to the molecular sieve precursor solution in is 1:2 to 1:10; more preferably, in the step (i), mass ratio of the fiber to the molecular sieve precursor solution is 1:2 to 1:5.

In view of the shortcomings of the prior art, the second technical problem to be solved by the present disclosure is to provide a method for synthesizing a molecular sieve/fiber composite material by using fibers as a scaffold for nucleation and crystal growth of molecular sieve, and a new template-free in-situ growth method, without adding any adhesive. This method has the characteristics of low cost, simple process and environmental friendliness.

The present disclosure adopts the following technical solutions:
The present disclosure provides a preparation method for a molecular sieve/fiber composite material as described above, including the following steps:
(i) prepare a molecular sieve precursor solution and mix it with the fiber;
(ii) the mixture of fiber and molecular sieve precursor solution in step (i) is processed with heat treatment to obtain a molecular sieve/fiber composite material.

In some embodiments, the molecular sieve precursor solution does not include a templating agent.

In some embodiments, in the step (ii), the temperature of the heat treatment is 60 to 220 °C, and the time of heat treatment is 4 to 240 h.

In some embodiments, in the step (i), mass ratio of the fiber to the molecular sieve precursor solution is 1:0.5 to 1:1000; preferably, in the step (i), mass ratio of the fiber to the molecular sieve precursor solution is 1:0.8 to 1:100; preferably, in the step (i), mass ratio of the fiber to the molecular sieve precursor solution is 1:1 to 1:50; preferably, in the step (i), mass ratio of the fiber to the molecular sieve precursor solution is 1:1.5 to 1:20; preferably, in the step (i), mass ratio of the fiber to the molecular sieve precursor solution in is 1:2 to 1:10; more preferably, in the step (i), mass ratio of the fiber to the molecular sieve precursor solution is 1:2 to 1:5.

In some embodiments, the molecular sieve is a mesoporous molecular sieve.

The prepared molecular sieve/fiber composite material includes molecular sieves and fibers. The molecular sieves are distributed on the fiber surface and directly contact the fiber surface, and the particle diameter D90 of the molecular sieves is 0.01 to 50 µm, the particle size D50 of the molecular sieves is 0.005 to 25 µm, and the molecular sieves are distributed uniformly on the fiber surface of the fiber.

The detection method of "uniform distribution of molecular sieves on the fiber surface" is: randomly taking n samples of the molecular sieve/fiber composite material at different locations and analyzing the content of the molecular sieve on the fiber surface, where n is a positive integer greater than or equal to 8. The coefficient of variation is also called the "standard deviation rate", which is the ratio of the standard deviation to the mean multiplied by 100%. The coefficient of variation is an absolute value that reflects the degree of dispersion of the data. The smaller the value of the coefficient of variation, the smaller the degree of dispersion of the data, indicating that the smaller the difference in the content of molecular sieves on the fiber surface, the more uniform the distribution of molecular sieves on the fiber surface. The coefficient of variation of the content of the molecular sieves in the n samples is ≤ 15%; preferably, the coefficient of variation of the content of the molecular sieves is ≤ 10%; preferably, the coefficient of variation of the content of the molecular sieves is ≤ 5%; preferably, the coefficient of variation of the content of the molecular sieves is ≤ 2%; preferably, the coefficient of variation of the content of the molecular sieves is ≤ 1%; preferably, the coefficient of variation of the content of the molecular sieves is ≤ 0.5%; preferably, the coefficient of variation of the content of the molecular sieves is ≤ 0.2%.

In some embodiments, the particle diameter D90 of the molecular sieve is 0.1 to 30 µm, and the particle diameter D50 of the molecular sieve is 0.05 to 15 µm; preferably, the particle diameter D90 of the molecular sieve is 0.5 to 20 µm, and the particle diameter D50 of the molecular sieve is 0.25 to 10 µm; preferably, the particle diameter D90 of the molecular sieve is 1 to 15 µm, and the particle diameter D50 of the molecular sieve is 0.5 to 8 µm; more preferably, the particle diameter D90 of the molecular sieve is 5 to 10 µm, and the particle diameter D50 of the molecular sieve is 2.5 to 5 µm.

In some embodiments, the adhesive content of the contact surface between the molecular sieve and the fiber is zero.

In some embodiments, the surface of molecular sieve contacted with the fiber is defined as inner surface, and the inner surface is a rough planar surface matched with the fiber surface; and growth-matched coupling is formed between the molecular sieve and the fiber on the inner surface of the molecular sieve, so that the molecular sieve has a strong binding strength with the fiber. The surface of molecular sieve uncontacted with the fiber is defined as outer surface, and the outer surface is a non-planar surface.

In some embodiments, the detection method for forming the growth-matched coupling is: for molecular sieve/fiber composite material, the retention rate of the molecular sieve on the fiber is greater than or equal to 90% under ultrasonic condition for 20 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, the detection method for forming the growth-matched coupling is: for molecular sieve/fiber composite material, the retention rate of the molecular sieve on the fiber is greater than or equal to 90% under ultrasonic condition for 40 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, the detection method for forming the growth-matched coupling is: for molecular sieve/fiber composite material, the retention rate of the molecular sieve on the fiber is greater than or equal to 90% under ultrasonic condition for 60 minutes or more; preferably, the retention rate is greater than or equal to 95%; more preferably, the retention rate is 100%, that is, the molecular sieve has a strong binding strength with the fiber, and the molecular sieve does not easily fall off the fiber surface.

In some embodiments, both the inner surface and outer surface are composed of molecular sieve nanoparticles.

The molecular sieve nanoparticles are particles formed by molecular sieve growing in a nanometer-scale size (2 to 500 nm).

In some embodiments, the average size of the molecular sieve nanoparticles of outer surface is larger than the average size of the molecular sieve nanoparticles of inner surface.

In some embodiments, the molecular sieves are independently dispersed on the fiber surface; and the molecular sieves are independently dispersed on the fiber surface so that the fiber maintains the original physical properties of flexibility and elasticity.

As an example, the aggregation of molecular sieves on the fiber surface (e.g. molecular sieves are distributed on the fiber surface in an agglomerated or lumpy structure) refer to the partial or full overlap of the molecular sieve microparticles and their nearest neighbor molecular sieve microparticles in space; that is, the minimum distance between the molecular sieve microparticles is less than one half of the sum of the particle size of the two molecular sieve microparticles (as shown in Figure 22(1), d < r₁+r₂).

Different from the aggregation of molecular sieve on the fiber surface, the independent dispersion means that the molecular sieve microparticles are dispersed on the fiber surface with a gap between each other, and the independent dispersion means the minimum distance between the molecular sieve microparticle and the nearest molecular sieve microparticle is greater than or equal to one half of the sum of the particle sizes of the two molecular sieve microparticles (as shown in Figure 22(2), d ≥ r₁+r₂), which indicates that there is a boundary between the adjacent molecular sieve microparticles.

In some embodiments, all the molecular sieves in the molecular sieve/fiber composite material are distributed on the fiber surface.

In some embodiments, the molecular sieve nanoparticles of outer surface are particles with corner angles.

In some embodiments, the molecular sieve nanoparticles of inner surface are particles without corner angles. The nanoparticles without corner angles make the inner surface of the molecular sieve match the fiber surface better, which is beneficial to the chemical combination of the molecular sieve and the fiber.

In some embodiments, the average size of the molecular sieve nanoparticles of inner surface is 2 to 100 nm; preferably, the average size of the molecular sieve nanoparticles of inner surface is 10 to 60 nm.

In some embodiments, the average size of the molecular sieve nanoparticles of outer surface is 50 to 500 nm; preferably, the average size of the molecular sieve nanoparticles of outer surface is 100 to 300 nm.

In some embodiments, the non-planar surface is composed of any one or combination of non-planar curves or non-planar lines. For example, the non-planar surface is composed of non-planar curves. Preferably, the non-planar surface is a spherical surface, and the spherical surface increases the effective area of contact with a substance. The spherical surface is made up of non-planar curves. In another example, the non-planar surface may be composed of non-planar curves and non-planar lines. In some embodiments, the non-planar line may be a polygonal line.

In some embodiments, the content of the molecular sieve accounts for 0.05 to 80 wt% of the molecular sieve/fiber composite material; preferably, the content of the molecular sieve accounts for 1 to 50 wt% of the molecular sieve/fiber composite material; preferably, the content of the molecular sieve accounts for 5 to 35 wt% of the molecular sieve/fiber composite material; preferably, the content of the molecular sieve accounts for 10 to 25 wt% of the molecular sieve/fiber composite material; more preferably, the content of the molecular sieve accounts for 15 to 20 wt% of the molecular sieve/fiber composite material.

In some embodiments, the molecular sieve may be selected from the group consisting of aluminosilicate molecular sieve, phosphate molecular sieve, borate molecular sieve, heteroatom molecular sieve, and combination thereof.

Further, the aluminosilicate molecular sieve can be selected from the group consisting of X-type molecular sieve, Y-type molecular sieve, A-type molecular sieve, ZSM-5 molecular sieve, chabazite, β-molecular sieve, mordenite, L-type molecular sieve, P-type molecular sieve, merlinoite, and combination thereof.

Further, the phosphate molecular sieve can be selected from the group consisting of AlPO₄-5 molecular sieve, AlPO₄-11 molecular sieve, SAPO-31 molecular sieve, SAPO-34 molecular sieve, SAPO-11, and combination thereof.

Further, the borate molecular sieve can be selected from the group consisting of BAC-1 molecular sieve, BAC-3 molecular sieve, BAC-10 molecular sieve, and combination thereof.

Further, the heteroatom molecular sieve is that a heteroatom element replaces part of silicon, aluminum, and phosphorus of the molecular sieve framework to form a molecular sieve containing other elements.

Further, the heteroatom element can be selected from the group consisting of transition metal elements in the fourth, fifth, sixth periods, and combination thereof.

In some embodiments, the molecular sieve is a molecular sieve after metal ion exchange. The metal ion is selected from the group consisting of strontium ion, calcium ion, magnesium ion, silver ion, zinc ion, barium ion, potassium ion, ammonium ion, copper ion, and combination thereof.

In some embodiments, the fiber is a polymer containing hydroxyl groups in a repeating unit.

Further, the fiber is selected from the group consisting of silk fiber, chitin fiber, rayon fiber, acetate fiber, carboxymethyl cellulose, bamboo fiber, cotton fiber, linen fiber, wool, wood fiber, lactide polymer fiber, glycolide polymer fiber, polyester fiber (abbreviated as PET), polyamide fiber (abbreviated as PA), polypropylene fiber (abbreviated as PP), polyethylene fiber (abbreviated as PE), polyvinyl chloride fiber (abbreviated as PVC), polyacrylonitrile fiber (abbreviated as acrylic fiber, artificial wool), viscose fiber, and combination thereof.

Further, the polyester fiber refers to a polyester obtained by polycondensation of a monomer having both a hydroxyl group and a carboxyl group, or a polyester obtained by polycondensation of an aliphatic dibasic acid and an aliphatic diol, or polyester or copolyester made from aliphatic lactone through ring-opening polymerization, and the molecular weight of the aliphatic polyester is 50,000 to 250,000. The polyester obtained by polycondensation of a monomer having both a hydroxyl group and a carboxyl group is a polylactic acid obtained by direct polycondensation of lactic acid; the polyester obtained by polycondensation of an aliphatic dibasic acid and an aliphatic diol is polybutylene succinate, polyhexanediol sebacate, polyethylene glycol succinate or polyhexyl succinate; polyester produced from ring-opening polymerization of aliphatic lactones is polylactic acid obtained by ring-opening polymerization of lactide, and polycaprolactone obtained by ring-opening polymerization of caprolactone; copolyester is poly(D,L-lactide-co-glycolide).

Further, the polyamide fiber refers to polyhexamethylene adipamide obtained by polycondensation of diamine and diacid, and the chemical structure formula of its long chain molecule is: H-[HN(CH₂)_{X}NHCO(CH₂)_{Y}CO]ₙ-OH; or obtained by polycondensation or ring-opening polymerization of caprolactam, the chemical structural formula of its long chain molecule is: H-[NH(CH₂)_{X}CO]n-OH.

A third object of the present disclosure is to provide a modified molecular sieve/fiber composite material, wherein the modified material comprising an additive, any forms of molecular sieve/fiber composite material as described above or any forms of molecular sieve/fiber composites made by the preparation method as described above.

In some embodiments, the additive is selected from the group consisting of metal, metal ion-containing compound, synthetic polymer compound, poorly soluble polysaccharide, protein, nucleic acid, pigment, antioxidant, mold inhibitor, detergent, surfactant, antibiotic, antibacterial agent, antimicrobial agent, anti-inflammatory agent, analgesic agent, antihistamine, and combination thereof.

In some embodiments, the synthetic polymer compounds are selected from the group consisting of polymer compounds prepared by self-addition polymerization reaction, polymer compounds obtained by polycondensation reaction, polyamide polymer compounds, synthetic rubber polymer compounds, and combination thereof.

In some embodiments, the proteins are selected from the group consisting of fibrin, collagen, oxidoreductase, transferase, hydrolase, lyase, isomerase, synthetase, and combination thereof.

In some embodiments, the transferase is selected from the group consisting of methyltransferase, aminotransferase, acetyltransferase, transferase, kinase, polymerase, and combination thereof.

In some embodiments, the hydrolase is selected from the group consisting of amylase, protease, lipase, phosphatase, glycosidase, and combination thereof.

In some embodiments, the lyase is selected from the group consisting of dehydratase, decarboxylase, carbonic anhydrase, aldolase, citrate synthase, and combination thereof.

In some embodiments, the synthetase is selected from the group consisting of glutamine synthetase, DNA ligase, biotin-dependent carboxylase, and combination thereof.

In some embodiments, the antibiotic is selected from the group consisting of quinolone antibiotic, β-lactam antibiotic, macrolide antibiotic, aminoglycoside antibiotic, and combination thereof.

In some embodiments, the poorly soluble polysaccharides may be natural non-chemically treated polymer polysaccharides, and the polymer polysaccharides are selected from the group consisting of cellulose, lignin, starch, chitosan, agarose, and combination thereof; the poorly soluble polysaccharides may also be chemically modified polymer polysaccharides, and the modified polymer polysaccharides are selected from the group consisting of carboxy-modified polymer polysaccharides, amino-modified polymer polysaccharides, thiol-modified polymer polysaccharides and combination thereof, and the polymer polysaccharides are selected from the group consisting of cellulose, lignin, starch, chitosan, agarose, and combination thereof. The poorly soluble polysaccharides according to the present disclosure may also be a mixture of natural non-chemically treated polymer polysaccharides and chemically modified polymer polysaccharides.

A fourth object of the present disclosure is to provide a compound, the compound comprising any one of the forms of molecular sieve/fiber composite material as described above or a molecular sieve/fiber composite material prepared by any of the forms of preparation methods as described above.

In some embodiments, the compound is a hemostatic material.

In some embodiments, the hemostatic material is a hemostatic textile.

Further, the hemostatic textile is selected from the group consisting of a hemostatic bandage, a hemostatic gauze, a hemostatic cloth, a hemostatic clothing, a hemostatic cotton, a hemostatic suture, a hemostatic paper, a hemostatic band-aid, and combination thereof.

Further, the hemostatic clothing is a material worn on the human body for protection or decoration.

Further, the hemostatic clothing is selected from the group consisting of hemostatic underwear, hemostatic vest, hemostatic cap, hemostatic pants, and combination thereof.

In some embodiments, the compound is an anti-radiation material.

Further, the anti-radiation material is selected from the group consisting of anti-radiation clothing, anti-radiation umbrella, anti-radiation cap, and combination thereof.

In some embodiments, the compound is an antibacterial material.

A fifth object of the present disclosure is to provide the application of the modified molecular sieve/fiber composite material in any form as described above in the fields of hemostasis, beauty, deodorization, sterilization, water purification, air purification, and radiation resistance.

A sixth object of the present disclosure is to provide the application of the compound in any form as described above in the fields of hemostasis, beauty, deodorization, sterilization, water purification, air purification, and radiation resistance.

The modified molecular sieve/fiber composite material or compound can be applied to the fields of hemostasis, beauty, deodorization, sterilization, water purification, air purification, and radiation resistance. For example, in the field of hemostasis, the modified molecular sieve/fiber composite material or compound can be made into hemostatic bandages, hemostatic gauze, hemostatic cloth, hemostatic clothing, etc. to solve the problems of emergency treatment for emergency accidents and trauma hemostasis during surgical operations on patients in hospitals. In the field of water purification, the characteristics of molecular sieves of the modified molecular sieve/fiber composite material or compound according to the present disclosure, which are firmly bonded to the fiber and maintain a large effective specific surface area of the molecular sieve, can be used in the purification process where the water is fully contacted with molecular sieve, and the harmful substances are absorbed to achieve the purpose of water purification.

The beneficial effects of the present disclosure are as follows.
1. For the first time, the present disclosure solves the problem of molecular sieve aggregation on the fiber surface in a molecular sieve/fiber composite material, and prepares a new molecular sieve/fiber composite material. The molecular sieves are uniformly distributed on the fiber surface of the fibers in a suitable size (the particle diameter D90 of the molecular sieve is 0.01 to 50 µm, and the particle diameter D50 of the molecular sieve is 0.005 to 30 µm), so that the overall performance of the composite material remains consistent. In the composite material of the present disclosure, the molecular sieves are distributed on the fiber surface and are in direct contact with the fiber surface, and a growth-matched coupling is formed between the molecular sieves and the fiber, and the molecular sieves and the fiber are firmly combined. Compared with traditional composite materials, the present disclosure does not require pretreatment of fibers and does not add an adhesive. The composite materials of the present disclosure solve the defects of agglomeration of molecular sieves on the fiber surface, low effective surface area of molecular sieves and blockage of molecular sieve channels; the molecular sieve/fiber composite material has high strength, elastic recovery ability, and dimensional stability, making the composite material strong and durable. The molecular sieve/fiber composite material of the present disclosure has a simple structure, low cost, strong stability, high repeatability of performance, and high practical efficiency.
2. The present disclosure provides a method for synthesizing a molecular sieve/fiber composite material by using fibers as a scaffold for nucleation and crystal growth of molecular sieve, and a template-free in-situ growth method. This method has the characteristics of low cost, simple process and environmental friendliness, and achieves good technical effects.
3. The modified molecular sieve/fiber composite material or compound according to the present disclosure can be used in the fields of hemostasis, beauty, deodorization, sterilization, water purification, air purification, and radiation resistance. In particular, its structural stability (the strong combination of molecular sieve and fiber) has a good application prospect in the medical industry, especially the hemostatic industry; the high effective surface area and material exchange capacity of the molecular sieves in the modified molecular sieve/fiber composite material or compound, make the material better used in adsorption, separation, catalysis and other fields.

### Brief Description of the Drawings

Figure 1 is a schematic diagram showing the destruction of a fiber structure caused by pretreatment of fiber in the prior art;
Figure 2 is a scanning electron microscope image of a molecular sieve/fiber composite material after pretreatment of fiber in the prior art, in which the molecular sieve is wrapped on the fiber surface in an agglomerated form (Journal of Porous Materials, 1996, 3 (3): 143-150);
Figure 3 is a scanning electron microscope image of a molecular sieve/fiber composite material after pretreatment of fiber in the prior art, in which the molecular sieve is wrapped on the fiber surface in an agglomerated or lumpy form (Cellulose, 2015, 22 (3): 1813-1827);
Figure 4 is a scanning electron microscope image of a molecular sieve/fiber composite material after pretreatment of fiber in the prior art, in which the molecular sieve is partially agglomerated and unevenly distributed on the fiber surface (Journal of Porous Materials, 1996, 3 (3): 143-150);
Figure 5 is a scanning electron microscope image of a molecular sieve/fiber composite material after pretreatment of fiber the prior art, in which a gap exists between the fiber and the molecular sieve (Journal of Porous Materials, 1996, 3 (3): 143-150);
Figure 6 is a scanning electron microscope image of a Na-LTA molecular sieve/nanocellulose fiber composite material bonded by polydiallyl dimethyl ammonium chloride in the prior art (ACS Appl. Mater. Interfaces 2016, 8, 3032-3040);
Figure 7 is a scanning electron microscope image of a NaY molecular sieve/fiber composite material bonded by a cationic and anionic polymer adhesive in the prior art (Microporous & Mesoporous Materials, 2011, 145 (1-3): 51-58);
Figure 8 is a schematic diagram of a molecular sieve/fiber composite material prepared by blend spinning in the prior art;
Figure 9 is a schematic diagram of molecular sieve/fiber composite material prepared by electrospun in the prior art (US Patent No. 7739452B2);
Figure 10 is a scanning electron microscope image of a molecular sieve/fiber composite material according to the present disclosure;
Figure 11 is a graph showing the content of molecular sieves on the fiber surface at 10 different locations of molecular sieve/fiber composite material according to the present disclosure;
Figure 12 is a scanning electron microscope image of fibers (a) and molecular sieves (b) corresponding to a hemostatic compound of the present disclosure, wherein the molecular sieves are obtained after removing the fibers from the hemostatic compound, and the fibers of Figure 12a are fibers in the hemostatic compound before the fibers are bonded with the molecular sieve (test parameter SU80100 3.0 kV; 9.9 mm).
Figure 13 shows a scanning electron microscope images of the inner surface (the contact surface between the molecular sieve and the fiber) and the outer surface of the molecular sieve of the molecular sieve/fiber composite material according to the present disclosure (test parameter SU80100 5.0 kV; 9.9 mm);
Figure 14 shows a statistical distribution diagram of particle sizes of nanoparticles on the inner surface (the contact surface between the molecular sieve and the fiber) and the outer surface of the molecular sieves of the molecular sieve/fiber composite material according to the present disclosure;
Figure 15 is a nitrogen adsorption and desorption isotherm diagram of a molecular sieve/fiber composite material according to the present disclosure, and the molecular sieve in the molecular sieve/fiber composite material is a mesoporous molecular sieve;
Figure 16 is a scanning electron microscope image of a molecular sieve of Comparative Example 1 (test parameter SU80100 5.0 kV; 9.9 mm);
Figure 17 is a schematic diagram showing the different binding strength of the molecular sieves and fibers of the molecular sieve/fiber composite material between the present disclosure and Comparative Example 2, with the influence of the growth-matched coupling between molecular sieves and fibers.
Figure 18 is a schematic diagram of a molecular sieve/fiber complex material bonded by an adhesive in the prior art. The fiber, the adhesive, and the molecular sieve form a sandwich-like structure, and the intermediate layer is an adhesive;
Figure 19 is a scanning electron microscope image of Combat Gauze commercialized by Z-Medica Co., Ltd. in Comparative Example 11;
Figure 20 is a picture of a clay/fiber complex material in an aqueous solution in the prior art;
Figure 21 is a graph of clay retention rate of clay/fiber complex material of a prior art in an aqueous solution under ultrasonic condition for different times;
Figure 22 is a schematic diagram of the positional relationship between two adjacent molecular sieve microparticles on the fiber surface of the hemostatic compound; wherein, schematic diagram (1) is the aggregation of molecular sieve on the fiber surface in the composite in the prior art, and schematic diagram (2) is the molecular sieve is independently dispersed on the fiber surface in the composite in the present disclosure.

### Detailed description of the preferred embodiment

The present disclosure is further described below with reference to the drawings and embodiments.

The "degree of ion exchange" is the ion exchange capacity of the compensation cations outside the molecular sieve framework and cations in the solution. The method for detecting the ion exchange capacity is: immersing a molecular sieve/fiber composite material in a 5M concentration strontium chloride, calcium chloride or magnesium chloride solution at room temperature for 12 hours to obtain a molecular sieve/fiber composite material after ion exchange, and measuring the degree of strontium ion, calcium ion or magnesium ion exchange of the molecular sieves of molecular sieve/fiber composite material after ion exchange.

"Effective specific surface area of molecular sieve" shows the specific surface area of the molecular sieve on the fiber surface in the molecular sieve/fiber composite material. The detection method of the effective specific surface area of the molecular sieve: the specific surface area of the molecular sieve/fiber composite material is analyzed by nitrogen isothermal adsorption and desorption, and the effective specific surface area of the molecular sieve = the specific surface area of the molecular sieve/fiber composite material - the specific surface area of the fiber.

Detection method of "content of molecular sieve on fiber surface": the mass fraction of molecular sieve on the fiber is analyzed using a thermogravimetric analyzer.

The detection method of "uniform distribution of molecular sieves on the fiber surface" is: randomly taking n samples of the molecular sieve/fiber composite material at different locations and analyzing the content of the molecular sieve on the fiber surface, where n is a positive integer greater than or equal to 8. The coefficient of variation is also called the "standard deviation rate", which is the ratio of the standard deviation to the mean multiplied by 100%. The coefficient of variation is an absolute value that reflects the degree of dispersion of the data. The smaller the value of the coefficient of variation, the smaller the degree of dispersion of the data, indicating that the smaller the difference in the content of molecular sieves on the fiber surface, the more uniform the distribution of molecular sieves on the fiber surface. The coefficient of variation of the content of the molecular sieves in the n samples is ≤ 15%, indicating that the molecular sieves are uniformly distributed on the fiber surface. Preferably, the coefficient of variation of the content of the molecular sieves is ≤ 10%, indicating that the molecular sieves are uniformly distributed on the fiber surface. Preferably, the coefficient of variation of the content of the molecular sieves is ≤ 5%, indicating that the molecular sieves are uniformly distributed on the fiber surface. Preferably, the coefficient of variation of the content of the molecular sieves is ≤ 2%, indicating that the molecular sieves are uniformly distributed on the fiber surface. Preferably, the coefficient of variation of the content of the molecular sieves is ≤ 1%, indicating that the molecular sieves are uniformly distributed on the fiber surface. Preferably, the coefficient of variation of the content of the molecular sieves is ≤ 0.5%, indicating that the molecular sieves are uniformly distributed on the fiber surface. Preferably, the coefficient of variation of the content of the molecular sieves is ≤ 0.2%, indicating that the molecular sieves are uniformly distributed on the fiber surface.

The detection method of the binding strength between the molecular sieve and the fiber is: putting the molecular sieve/fiber composite material in deionized water under ultrasonic condition for 20 min or more, and analyzing the content of the molecular sieve on the fiber surface by using a thermogravimetric analyzer. The retention rate on the fiber, the retention rate = (content of the molecular sieve on the fiber surface before the ultrasound - content of the molecular sieve on the fiber surface after the ultrasound) × 100% / content of the molecular sieve on the fiber surface before the ultrasound. If the retention rate is greater than or equal to 90%, it indicates that molecular sieve and fiber form a growth-matched coupling, and the molecular sieve is firmly bonded to the fiber.

The detection methods of D50 and D90 are: using scanning electron microscope to observe the molecular sieve microparticles on the surface of the molecular sieve/fiber composite material, and carrying out statistical analysis of particle size. D50 refers to the particle size corresponding to the cumulative particle size distribution percentage of the molecular sieve microparticles reaching 50%. D90 refers to the particle size corresponding to the cumulative particle size distribution percentage of the molecular sieve microparticles reaching 90%.

Detection method of hemostatic function: The hemostatic function of molecular sieve/fiber composite material is evaluated by using a rabbit femoral artery lethal model. The specific steps are as follows: (1) before the experiment, white rabbits were anesthetized with sodium pentobarbital intravenously (45 mg/kg); their limbs and head were fixed, and supine on the experimental table; part of the hair was removed to expose the right groin of the hind limb. (2) Then, the femoral skin and muscle were cut longitudinally to expose the femoral artery, and the femoral artery was partially cut off (about half of the circumference). After the femoral artery was allowed to squirt freely for 30 seconds, the blood at the wound was cleaned with cotton gauze, and then the hemostatic material was quickly pressed to the wound. After pressing for 60 seconds, the hemostatic material is lifted up slightly every 10 seconds to observe the coagulation of the injured part and the coagulation time is recorded. Infrared thermometers are used to detect changes in wound temperature (before and after using hemostatic material). (3) After hemostasis, observe the wound and suture the wound. The survival of the animals is observed for 2 hours after hemostasis. The survival rate = (total number of experimental white rabbits-number of deaths of white rabbits observed for 2 hours after hemostasis) × 100% / total number of experimental white rabbits, wherein the number of experimental white rabbits in each group is n, n is a positive integer greater than or equal to 6. (4) The difference in weight of the hemostatic material before and after use was recorded as the amount of blood loss during wound hemostasis.

### Example 1

The preparation method of the Y-type molecular sieve/cotton fiber composite material of the present disclosure includes the following steps:
(i) A molecular sieve precursor solution was prepared, and a starting material was composed of 10Na₂O : Al₂O₃ : 9SiO₂ : 200H₂O in a molar ratio to synthesize a molecular sieve precursor solution. The molecular sieve precursor solution was mixed with cotton fiber, and the mass ratio of the cotton fiber and the molecular sieve precursor solution is 1:20.
(ii) The cotton fiber and the homogeneously-mixed molecular sieve precursor solution were heat-treated at 100 °C for 24 h to obtain a Y-type molecular sieve/cotton fiber composite material.

Ten samples of the prepared Y-type molecular sieve/cotton fiber composite material were randomly taken at different locations, and the content of the Y-type molecular sieve on the fiber surface was analyzed by a thermogravimetric analyzer. The content of molecular sieve on the fiber in the ten samples was 25 wt%, 24.9 wt%, 25.1 wt%, 25.2 wt%, 25 wt%, 25 wt%, 24.9 wt%, 25 wt%, 25.1 wt%, 24.9 wt%. The average content of molecular sieves on the fibers in the ten samples was 25 wt%, the standard deviation of the samples is 0.1 wt%, and the coefficient of variation is 0.4%, which indicates that the Y-type molecular sieve is uniformly distributed on the fiber surface.

The prepared Y-type molecular sieve/cotton fiber composite material was observed with a scanning electron microscope. Hemispherical molecular sieves with an average particle size of 5 µm are independently dispersed on the fiber surface, as shown in Figure 10. The molecular sieves microparticles of the molecular sieve/fiber composite material are observed with a scanning electron microscope, and are performed statistical analysis of particle size to obtain a particle diameter D90 value of 25 µm and a particle diameter D50 value of 5 µm. The molecular sieves are obtained after removing the fibers by calcination. The inner surface of the molecular sieves in contact with the fiber is planar surface (caused by tight binding with the fiber), and the outer surface is spherical surface. The planar surface of the inner surface of the molecular sieves is a rough surface (Figure 12). The outer surface of the molecular sieve is composed of nanoparticles with corner angles, and the inner surface (the contact surface with the fiber) is composed of nanoparticles without corner angles (Figure 13). The nanoparticles without corner angles make the inner surface of the molecular sieve match the fiber surface better, which is beneficial to the combination of the molecular sieve and the fiber. The average size of nanoparticles of the inner surface (148 nm) is significantly smaller than that of the outer surface (61 nm), and small-sized particles are more conducive to binding with fibers tightly (Figure 14). The detection method of the binding strength between the molecular sieve and the fiber: the molecular sieve/fiber composite material is under ultrasonic condition in deionized water for 20 min, and the content of the Y-type molecular sieves on the fiber surface is analyzed by using a thermogravimetric analyzer. It is found that the content of the molecular sieve on the fiber surface is the same before and after ultrasonic condition. It shows that the retention rate of molecular sieve on the fiber is 100%, which indicates that the growth-matched coupling is formed between the molecular sieve and the fiber, and the molecular sieve is firmly bonded to the fiber. The molecular sieve in the Y-type molecular sieve/cotton fiber composite material was analyzed by nitrogen isothermal adsorption and desorption, and a hysteresis loop was found in the isothermal adsorption curve, indicating that the molecular sieve has a mesoporous structure (Figure 15). Using the method for detecting the effective specific surface area of the molecular sieve as described above, the effective specific surface area of the molecular sieve in the Y-type molecular sieve/cotton fiber composite material prepared in this embodiment was measured to be 490 m²g⁻¹. Using the method for detecting the ion exchange capacity of the molecular sieve in the Y-type molecular sieve/cotton fiber composite material, the degree of calcium ion exchange is 99.9%, the degree of magnesium ion exchange is 97%, and the degree of strontium ion exchange is 90%. Comparative Example 1
(1) A molecular sieve precursor solution was prepared, and a starting material was composed of 10Na₂O : Al₂O₃ : 9SiO₂ : 200H₂O in a molar ratio to synthesize a molecular sieve precursor solution.
(2) The molecular sieve precursor solution was heat-treated at 100 °C for 24 h to obtain a Y-type molecular sieve.

The effective specific surface area of the Y-type molecular sieve was 490 m²g⁻¹, the degree of calcium ion exchange was 99.9%, the degree of magnesium ion exchange was 97%, and the degree of strontium ion exchange was 90%.

The effective specific surface area and ion exchange capacity of the above Y-type molecular sieve are used as reference values to evaluate the performance of the molecular sieve to the fiber surface in the Comparative Examples described below. The difference between this Comparative Example 1 and Example 1 is that only the Y-type molecular sieve is synthesized without adding fibers (the traditional solution growth method). Using a scanning electron microscope (Figure 16), the synthesized molecular sieve is a complete microsphere composed of nanoparticles, and there is no rough planar surface (inner surface) in contact with the fibers compared with Example 1.

### Comparative Example 2

(1) A molecular sieve precursor solution was prepared, and a starting material was composed of 10Na₂O : Al₂O₃ : 9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) The above Y-type molecular sieve was added with deionized water to uniformly disperse the Y-type molecular sieve in an aqueous solution.
(4) Immerse the cotton fiber in the solution prepared in step (3) and soak for 30 min.
(5) Dry at 65 °C to obtain a Y-type molecular sieve/cotton fiber complex material (impregnation method).

The difference between this Comparative Example and Example 1 is that only the Y-type molecular sieve is synthesized without adding fibers (the traditional solution growth method). Using a scanning electron microscope, the synthesized molecular sieve is a complete microsphere composed of nanoparticles, and there is no rough planar surface (inner surface) in contact with the fibers compared with Example 1. Therefore, there is no growth-matched coupling between the molecular sieve and the fiber surface. The binding strength between the molecular sieve and the fiber was measured. The Y-type molecular sieve/cotton fiber complex material (impregnation method) was under the ultrasonic condition for 20 min, the retention rate of the molecular sieve on the fiber was 5%, indicating that the molecular sieve of Y-type molecular sieve/cotton fiber complex material (impregnation method) has a weak binding effect with the fiber, and the molecular sieve easily falls off (Figure 17).

### Comparative Example 3

(1) A molecular sieve precursor solution was prepared, and a starting material was composed of 10Na₂O : Al₂O₃ : 9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) The above Y-type molecular sieve was added with deionized water to uniformly disperse the Y-type molecular sieve in an aqueous solution.
(4) Spray the solution prepared in step (3) on cotton fibers
(5) Dry at 65 °C to obtain a Y-type molecular sieve/cotton fiber complex material (spray method).

The difference between this Comparative Example and Example 1 is that the synthesized molecular sieve is sprayed onto cotton fibers. Using a scanning electron microscope, there is no rough planar surface (inner surface) in contact with the fibers compared with Example 1. Therefore, there is no growth-matched coupling between the molecular sieve and the fiber surface. The binding strength between the molecular sieve and the fiber was measured. The molecular sieve/fiber complex material was under the ultrasonic condition for 20 min, the retention rate of the molecular sieve on the fiber was 2%, indicating that the molecular sieve of Y-type molecular sieve/cotton fiber complex material (spray method) has a weak binding effect with the fiber, and the molecular sieve easily falls off.

### Comparative Example 4

Refer to references for experimental steps (ACS Appl Mater Interfaces, 2016, 8(5):3032-3040).
(1) A molecular sieve precursor solution was prepared, and a starting material was composed of 10Na₂O : Al₂O₃ :9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) The above Y-type molecular sieve was added with deionized water to uniformly disperse the Y-type molecular sieve in an aqueous solution.
(4) Cotton fibers were immersed in a 0.5 wt% polydiallyl dimethyl ammonium chloride (polyDADMAC) aqueous solution at 60 °C for 30 minutes to achieve adsorption of Y-type molecular sieves (polyDADMAC is an adhesive 1).
(5) Dry at 65 °C to obtain a Y-type molecular sieve/cotton fiber complex material (including adhesive 1).

The difference between this Comparative Example and Example 1 is that the synthesized molecular sieve is bonded to cotton fibers through an adhesive. After detection of scanning electron microscope, there is no rough planar surface (inner surface) in contact with the fiber, so there is no growth-matched coupling. The binding strength between the molecular sieve and the fiber was measured. The retention rate of the molecular sieve on the fiber was 50% under ultrasonic condition for 20 min, indicating that the molecular sieve has a weak binding strength with the fiber, and the molecular sieve in the Y-type molecular sieve/cotton fiber complex material (including adhesive 1) easily falls off. After detection of scanning electron microscope, the molecular sieve was unevenly distributed on the fiber surface, and there was agglomeration of the molecular sieve. After testing, with the addition of adhesive, the effective specific surface area of the molecular sieve became 320 m²g⁻¹, the degree of calcium ion exchange became 75.9%, the degree of magnesium ion exchange became 57%, and the degree of strontium ion exchange became 50%. The complex material with added adhesive reduces the effective contact area between the molecular sieve and the reaction system, and reduces the ion exchange and pore substance exchange capacity of the molecular sieve.

Ten samples of the prepared Y-type molecular sieve/cotton fiber complex material (including adhesive 1) were randomly taken at different locations, and the content of the Y-type molecular sieve on the fiber surface was analyzed. The average content of molecular sieves on the fibers in the ten samples was 25 wt%, the standard deviation of the samples is 10 wt%, and the coefficient of variation is 40%, which indicates that the Y-type molecular sieve is unevenly distributed on the fiber surface.

### Comparative Example 5

Refer to references for experimental steps (Colloids & Surfaces B Biointerfaces, 2018, 165:199).
(1) A molecular sieve precursor solution was prepared, and a starting material was composed of 10Na₂O : Al₂O₃ :9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) The above Y-type molecular sieve was dispersed in a polymeric N-halamine precursor water/ethanol solution (polymeric N-halamine precursor is an adhesive 2).
(4) The solution prepared in the step (3) was sprayed on cotton fibers
(5) Dry at 65 °C to obtain a Y-type molecular sieve/cotton fiber complex material (including adhesive 2).

The difference between this Comparative Example and Example 1 is that the molecular sieves with an adhesive were sprayed onto cotton fibers. After detection of scanning electron microscope, there is no rough planar surface (inner surface) in contact with the fiber, so there is no growth-matched coupling. The binding strength between the molecular sieve and the fiber was measured. The retention rate of the molecular sieve on the fiber was 41% under ultrasonic condition for 20 min, indicating that the molecular sieve has a weak binding strength with the fiber, and the molecular sieve in the Y-type molecular sieve/cotton fiber complex material (including adhesive 2) easily falls off. After detection of scanning electron microscope, the molecular sieve was unevenly distributed on the fiber surface, and there was agglomeration of the molecular sieve. After testing, with the addition of adhesive, the effective specific surface area of the molecular sieve became 256 m²g⁻¹, the degree of calcium ion exchange became 65.9%, the degree of magnesium ion exchange became 47%, and the degree of strontium ion exchange became 42%. The complex material with added adhesive reduces the effective contact area between the molecular sieve and the reaction system, and reduces the ion exchange and pore substance exchange capacity of the molecular sieve.

Ten samples of the prepared Y-type molecular sieve/cotton fiber complex material (including adhesive 2) were randomly taken at different locations, and the content of the Y-type molecular sieve on the fiber surface was analyzed. The average content of molecular sieves on the fibers in the ten samples was 25 wt%, the standard deviation of the samples is 4 wt%, and the coefficient of variation is 16%, which indicates that the Y-type molecular sieve is unevenly distributed on the fiber surface.

### Comparative Example 6

Refer to references for experimental steps (Key Engineering Materials, 2006, 317-318:777-780).
(1) A molecular sieve precursor solution was prepared, and a starting material was composed of 10Na₂O : Al₂O₃ :9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) The Y-type molecular sieve sample was dispersed in a silica sol-based inorganic adhesive (adhesive 3) solution to obtain a slurry of a molecular sieve and adhesive mixture.
(4) The prepared slurry in the step (3) was coated on cotton fibers, and then kept at room temperature for 1 h, and then kept at 100 °C for 1 h. The fibers were completely dried to obtain a Y-type molecular sieve/cotton fiber complex material (including adhesive 3).

The difference between this Comparative Example and Example 1 is that the molecular sieves with a silica sol-based adhesive were coated on the cotton fibers. After detection of scanning electron microscope, there is no rough planar surface (inner surface) in contact with the fiber, so there is no growth-matched coupling. The binding strength between the molecular sieve and the fiber was measured. The retention rate of the molecular sieve on the fiber was 46% under ultrasonic condition for 20 min, indicating that the molecular sieve has a weak binding strength with the fiber, and the molecular sieve in the Y-type molecular sieve/cotton fiber complex material (including adhesive 3) easily falls off. After detection of scanning electron microscope, the molecular sieve was unevenly distributed on the fiber surface, and there was agglomeration of the molecular sieve. After testing, with the addition of adhesive, the effective specific surface area of the molecular sieve became 246 m²g⁻¹, the degree of calcium ion exchange became 55.9%, the degree of magnesium ion exchange became 57%, and the degree of strontium ion exchange became 40%. The complex material with added adhesive reduces the effective contact area between the molecular sieve and the reaction system, and reduces the ion exchange and pore substance exchange capacity of the molecular sieve.

Ten samples of the prepared Y-type molecular sieve/cotton fiber complex material (including adhesive 3) were randomly taken at different locations, and the content of the Y-type molecular sieve on the fiber surface was analyzed. The average content of molecular sieves on the fibers in the ten samples was 25 wt%, the standard deviation of the samples is 8.5 wt%, and the coefficient of variation is 34%, which indicates that the Y-type molecular sieve is unevenly distributed on the fiber surface.

### Comparative Example 7

Refer to references for experimental steps (Journal of Porous Materials, 1996, 3(3): 143-150).
(1) The fibers were chemically pretreated. The fibers were first treated with ether for 20 minutes and sonicated in distilled water for 10 minutes.
(2) A molecular sieve precursor solution was prepared, and a starting material was composed of 7.5Na₂O : Al₂O₃ : 10SiO₂ : 230H₂O in a molar ratio to synthesize a molecular sieve precursor solution, followed by magnetic stirring for 1 h and standing at room temperature for 24 h. The molecular sieve precursor solution was mixed with pretreated cotton fibers.
(3) The pretreated cotton fiber and the homogeneously-mixed molecular sieve precursor solution were heat-treated at 100 °C for 6 h to obtain a Y-type molecular sieve/cotton fiber complex material (pretreatment of fiber).

The difference between this Comparative Example and Example 1 is that the fiber is pretreated, but the structure of the fiber itself is seriously damaged, which affects the characteristics such as the flexibility and elasticity of the fiber, and the fiber becomes brittle and hard. Therefore, the advantages of fiber as a carrier cannot be fully utilized. After detection by a scanning electron microscope, the molecular sieve was wrapped in the outer layer of the fiber, and there was still a gap between the fiber and the molecular sieve, indicating that this technology cannot tightly combine molecular sieve and fiber. Compared with Example 1, there is no rough planar surface (inner surface) in contact with the fiber, so there is no growth-matched coupling. The binding strength between the molecular sieve and the fiber was measured. The retention rate of the molecular sieve on the fiber was 63% under ultrasonic condition for 20 min, indicating that the molecular sieve has a weak binding strength with the fiber, and the molecular sieve in the Y-type molecular sieve/cotton fiber complex material (pretreatment of fiber) easily falls off. After testing, the agglomeration of molecular sieve makes the effective specific surface area of the molecular sieve to become 346 m²g⁻¹, the degree of calcium ion exchange become 53%, the degree of magnesium ion exchange become 52%, and the degree of strontium ion exchange become 42%, which greatly reduces the effective contact area between the effective molecular sieve and the reaction system, and reduces the ion exchange and pore substance exchange capacity of the molecular sieve.

Ten samples of the prepared Y-type molecular sieve/cotton fiber complex material (pretreatment of fiber) were randomly taken at different locations, and the content of the Y-type molecular sieve on the fiber surface was analyzed. The average content of molecular sieves on the fibers in the ten samples was 25 wt%, the standard deviation of the samples is 9 wt%, and the coefficient of variation is 36%, which indicates that the Y-type molecular sieve is unevenly distributed on the fiber surface.

### Comparative Example 8

Refer to Chinese patent CN104888267A for experimental steps.
(1) A molecular sieve precursor solution was prepared, and a starting material was composed of 10Na₂O : Al₂O₃ : 9SiO₂ : 200H₂O in a molar ratio to synthesize a molecular sieve precursor solution.
(2) The molecular sieve precursor solution was heat-treated at 100 °C for 24 h to obtain a Y-type molecular sieve.
(3) Prepare polyurethane urea stock solution.
(4) The Y-type molecular sieve is ground in a dimethylacetamide solvent to obtain a Y-type molecular sieve solution.
(5) The polyurethane urea stock solution and the Y-type molecular sieve solution are simultaneously placed in a reaction container, and spandex fibers are prepared through a dry spinning process, and finally woven into a Y-type molecular sieve/spandex fiber complex material (blend spinning).

The difference between this Comparative Example and Example 1 is that the Y-type molecular sieve is blended and spun into the fiber. After detection by a scanning electron microscope, molecular sieve and fiber were simply physically mixed, and there was no growth-matched coupling. After testing, this method makes the effective specific surface area of the molecular sieve become 126 m²g⁻¹, the degree of calcium ion exchange become 45.9%, the degree of magnesium ion exchange become 27%, and the degree of strontium ion exchange become 12%. The blend spinning method is used to prepare molecular sieve/fiber complex material, which greatly reduces the effective contact area between the effective molecular sieve and the reaction system, and reduces the ion exchange and pore substance exchange capacity of the molecular sieve.

### Comparative Example 9

(i) A molecular sieve precursor solution was prepared, and a starting material was composed of 10Na₂O : Al₂O₃ : 9SiO₂ : 200H₂O in a molar ratio to synthesize a molecular sieve precursor solution. The molecular sieve precursor solution is mixed with cotton fiber, and the mass ratio of the cotton fiber and the molecular sieve precursor solution is 1:0.3.
(ii) The cotton fiber and the homogeneously-mixed molecular sieve precursor solution were heat-treated at 100 °C for 24 h to obtain a Y-type molecular sieve/cotton fiber complex material. The content of Y-type molecular sieve was 90 wt%.

The difference between this Comparative Example and Example 1 is that the content of the Y-type molecular sieves is different. The content of the Y-type molecular sieves of this Comparative Example is greater than 80 wt%. After detection by a scanning electron microscope, the molecular sieves are clumped and wrapped on the fiber surface. The molecular sieves are not independently dispersed on the fiber surface, resulting in fiber stiffening. After testing, the agglomeration of molecular sieves makes the effective specific surface area of the molecular sieve become 346 m²g⁻¹, the degree of calcium ion exchange become 53%, the degree of magnesium ion exchange become 52%, and the degree of strontium ion exchange become 42%. Both the effective specific surface area and ion exchange capacity are significantly reduced.

### Example 2

The preparation method of the chabazite/cotton fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with cotton fiber, and the mass ratio of the cotton fiber and the molecular sieve precursor solution is 1:0.5.
(ii) The cotton fiber and the homogeneously-mixed molecular sieve precursor solution were heat-treated at 80 °C for 36 h to obtain a chabazite/cotton fiber composite material.

Ten samples of the prepared chabazite/cotton fiber composite material were randomly taken at different locations, and the content of the chabazite on the fiber surface was analyzed. The average content of molecular sieves on the fibers in the ten samples was 25 wt%, the standard deviation of the samples is 2.5 wt%, and the coefficient of variation is 10%, which indicates that the chabazite is uniformly distributed on the fiber surface.

### Example 3

The preparation method of the X-type molecular sieve/silk fiber composite material of the present disclosure includes the following steps:
(i) A molecular sieve precursor solution was prepared, and a starting material was composed of 5.5Na₂O : 1.65K₂O : Al₂O₃ : 2.2SiO₂ : 122H₂O in a molar ratio to synthesize a molecular sieve precursor solution. The molecular sieve precursor solution was mixed with silk fiber, and the mass ratio of the silk fiber and the molecular sieve precursor solution is 1:10.
(ii) The silk fiber and the homogeneously-mixed molecular sieve precursor solution were heat-treated at 100 °C for 12 h to obtain a X-type molecular sieve/silk fiber composite material.

Eight samples of the prepared X-type molecular sieve/silk fiber composite material were randomly taken at different locations, and the content of the X-type molecular sieve on the fiber surface was analyzed. The average content of molecular sieves on the fibers in the eight samples was 15 wt%, the standard deviation of the samples is 1.5 wt%, and the coefficient of variation is 10%, which indicates that the X-type molecular sieve is uniformly distributed on the fiber surface.

### Example 4

The preparation method of the A-type molecular sieve/polyester fiber composite material of the present disclosure includes the following steps:
(i) A molecular sieve precursor solution was prepared, and a starting material was composed of 3Na₂O : Al₂O₃ : 2SiO₂ : 120H₂O in a molar ratio to synthesize a molecular sieve precursor solution. The molecular sieve precursor solution was mixed with polyester fiber, and the mass ratio of the polyester fiber and the molecular sieve precursor solution is 1:50.
(ii) The polyester fiber and the homogeneously-mixed molecular sieve precursor solution were heat-treated at 100 °C for 4 h to obtain a A-type molecular sieve/polyester fiber composite material.

Ten samples of the prepared A-type molecular sieve/polyester fiber composite material were randomly taken at different locations, and the content of the A-type molecular sieve on the fiber surface was analyzed. The average content of molecular sieves on the fibers in the ten samples was 50 wt%, the standard deviation of the samples is 7.5 wt%, and the coefficient of variation is 15%, which indicates that the A-type molecular sieve is uniformly distributed on the fiber surface.

### Example 5

The preparation method of the ZSM-5 molecular sieve/polypropylene fiber composite material of the present disclosure includes the following steps:
(i) A molecular sieve precursor solution was prepared, and a starting material was composed of 3.5Na₂O : Al₂O₃ : 28SiO₂ : 900H₂O in a molar ratio to synthesize a molecular sieve precursor solution. The molecular sieve precursor solution was mixed with polypropylene fiber, and the mass ratio of the polypropylene fiber and the molecular sieve precursor solution is 1:200.
(ii) The polypropylene fiber and the homogeneously-mixed molecular sieve precursor solution were heat-treated at 180°C for 42 h to obtain a ZSM-5 molecular sieve/polypropylene fiber composite material.

Ten samples of the prepared ZSM-5 molecular sieve/polypropylene fiber composite material were randomly taken at different locations, and the content of the ZSM-5 molecular sieve on the fiber surface was analyzed. The average content of molecular sieves on the fibers in the ten samples was 30 wt%, the standard deviation of the samples is 1.5 wt%, and the coefficient of variation is 5%, which indicates that the ZSM-5 molecular sieve is uniformly distributed on the fiber surface.

### Example 6

The preparation method of the β-molecular sieve/rayon fiber composite material of the present disclosure includes the following steps:
(i) A molecular sieve precursor solution was prepared, and a starting material was composed of 2Na₂O : 1.1K₂O Al₂O₃ : 50SiO₂ : 750H₂O : 3HCl in a molar ratio to synthesize a molecular sieve precursor solution. The molecular sieve precursor solution was mixed with rayon fiber, and the mass ratio of the rayon fiber and the molecular sieve precursor solution is 1:100.
(ii) The rayon fiber and the homogeneously-mixed molecular sieve precursor solution were heat-treated at 135°C for 25 h to obtain a β-molecular sieve/rayon fiber composite material.

Eight samples of the prepared β-molecular sieve/rayon fiber composite material were randomly taken at different locations, and the content of the β-molecular sieve on the fiber surface was analyzed. The average content of molecular sieves on the fibers in the eight samples was 25 wt%, the standard deviation of the samples is 2 wt%, and the coefficient of variation is 8%, which indicates that the β-molecular sieve is uniformly distributed on the fiber surface.

### Example 7

The preparation method of the mordenite/acetate fiber composite material of the present disclosure includes the following steps:
(i) A molecular sieve precursor solution was prepared, and a starting material was composed of 5.5Na₂O : Al₂O₃ : 30SiO₂ : 810H₂O in a molar ratio to synthesize a molecular sieve precursor solution. The molecular sieve precursor solution was mixed with acetate fiber, and the mass ratio of the acetate fiber and the molecular sieve precursor solution is 1:300.
(ii) The acetate fiber and the homogeneously-mixed molecular sieve precursor solution were heat-treated at 170°C for 24 h to obtain a mordenite/acetate fiber composite material.

Ten samples of the prepared mordenite/acetate fiber composite material were randomly taken at different locations, and the content of the mordenite on the fiber surface was analyzed. The average content of molecular sieves on the fibers in the ten samples was 35 wt%, the standard deviation of the samples is 5.25 wt%, and the coefficient of variation is 15%, which indicates that the mordenite is uniformly distributed on the fiber surface.

### Example 8

The preparation method of the L-type molecular sieve/carboxymethyl cellulose composite material of the present disclosure includes the following steps:
(i) A molecular sieve precursor solution was prepared, and a starting material was composed of 2.5K₂O : Al₂O₃ : 12SiO₂ : 155H₂O in a molar ratio to synthesize a molecular sieve precursor solution. The molecular sieve precursor solution was mixed with carboxymethyl cellulose, and the mass ratio of the carboxymethyl cellulose and the molecular sieve precursor solution is 1:1.
(ii) The carboxymethyl cellulose and the homogeneously-mixed molecular sieve precursor solution were heat-treated at 220 °C for 50 h to obtain a L-type molecular sieve/carboxymethyl cellulose composite material.

Ten samples of the prepared L-type molecular sieve/carboxymethyl cellulose composite material were randomly taken at different locations, and the content of the L-type molecular sieve on the fiber surface was analyzed. The average content of molecular sieves on the fibers in the ten samples was 10 wt%, the standard deviation of the samples is 0.2 wt%, and the coefficient of variation is 2%, which indicates that the L-type molecular sieve is uniformly distributed on the fiber surface.

### Example 9

The preparation method of the P-type molecular sieve/bamboo fiber composite material of the present disclosure includes the following steps:
(i) A molecular sieve precursor solution was prepared, and a starting material was composed of 10Na₂O : Al₂O₃ : 9SiO₂ : 400H₂O in a molar ratio to synthesize a molecular sieve precursor solution. The molecular sieve precursor solution was mixed with bamboo fiber, and the mass ratio of the bamboo fiber and the molecular sieve precursor solution is 1:2.
(ii) The bamboo fiber and the homogeneously-mixed molecular sieve precursor solution were heat-treated at 150 °C for 96 h to obtain a P-type molecular sieve/bamboo fiber composite material.

Twenty samples of the prepared P-type molecular sieve/bamboo fiber composite material were randomly taken at different locations, and the content of the P-type molecular sieve on the fiber surface was analyzed. The average content of molecular sieves on the fibers in the twenty samples was 80 wt%, the standard deviation of the samples is 4 wt%, and the coefficient of variation is 5%, which indicates that the P-type molecular sieve is uniformly distributed on the fiber surface.

### Example 10

The preparation method of the merlinoite/linen fiber composite material of the present disclosure includes the following steps:
(i) A molecular sieve precursor solution was prepared, and a starting material was composed of 10Na₂O : Al₂O₃ : 9SiO₂ : 320H₂O in a molar ratio to synthesize a molecular sieve precursor solution. The molecular sieve precursor solution was mixed with linen fiber, and the mass ratio of the linen fiber and the molecular sieve precursor solution is 1:1000.
(ii) The linen fiber and the homogeneously-mixed molecular sieve precursor solution were heat-treated at 120 °C for 24 h to obtain a merlinoite/linen fiber composite material.

Fifteen samples of the prepared merlinoite/linen fiber composite material were randomly taken at different locations, and the content of the merlinoite on the fiber surface was analyzed. The average content of molecular sieves on the fibers in the fifteen samples was 30 wt%, the standard deviation of the samples is 0.3 wt%, and the coefficient of variation is 1%, which indicates that the merlinoite is uniformly distributed on the fiber surface.

### Example 11

The preparation method of the X-type molecular sieve/wool composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with wool, and the mass ratio of the wool and the molecular sieve precursor solution is 1:20.
(ii) The wool and the homogeneously-mixed molecular sieve precursor solution were heat-treated at 60 °C for 16 h to obtain a X-type molecular sieve/wool composite material.

Fifteen samples of the prepared X-type molecular sieve/wool composite material were randomly taken at different locations, and the content of the X-type molecular sieve on the fiber surface was analyzed. The average content of molecular sieves on the fibers in the fifteen samples was 27 wt%, the standard deviation of the samples is 2.1 wt%, and the coefficient of variation is 7.8%, which indicates that the X-type molecular sieve is uniformly distributed on the fiber surface.

### Example 12

The preparation method of the X-type molecular sieve/wood fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with wood fiber, and the mass ratio of the wood fiber and the molecular sieve precursor solution is 1:5. The content of X-type molecular sieve was 42 wt%.
(ii) The wood fiber and the homogeneously-mixed molecular sieve precursor solution were heat-treated at 90 °C for 24 h to obtain a X-type molecular sieve/wood fiber composite material.

Fifteen samples of the prepared X-type molecular sieve/wood fiber composite material were randomly taken at different locations, and the content of the X-type molecular sieve on the fiber surface was analyzed. The average content of molecular sieves on the fibers in the fifteen samples was 42 wt%, the standard deviation of the samples is 2.1 wt%, and the coefficient of variation is 5%, which indicates that the X-type molecular sieve is uniformly distributed on the fiber surface.

### Example 13

The preparation method of the X-type molecular sieve/lactide polymer fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with lactide polymer fiber, and the mass ratio of the lactide polymer fiber and the molecular sieve precursor solution is 1:50.
(ii) The lactide polymer fiber and the homogeneously-mixed molecular sieve precursor solution were heat-treated at 90 °C for 30 h to obtain a X-type molecular sieve/lactide polymer fiber composite material. The content of X-type molecular sieve was 26 wt%.

Fifteen samples of the prepared X-type molecular sieve/lactide polymer fiber composite material were randomly taken at different locations, and the content of the X-type molecular sieve on the fiber surface was analyzed. The average content of molecular sieves on the fibers in the fifteen samples was 26 wt%, the standard deviation of the samples is 1.1 wt%, and the coefficient of variation is 4.2%, which indicates that the X-type molecular sieve is uniformly distributed on the fiber surface.

### Example 14

The preparation method of the X-type molecular sieve/glycolide polymer fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with glycolide polymer fiber, and the mass ratio of the glycolide polymer fiber and the molecular sieve precursor solution is 1:200.
(ii) The glycolide polymer fiber and the homogeneously-mixed molecular sieve precursor solution were heat-treated at 120 °C for 24 h to obtain a X-type molecular sieve/glycolide polymer fiber composite material. The content of X-type molecular sieve was 37 wt%.

Fifteen samples of the prepared X-type molecular sieve/glycolide polymer fiber composite material were randomly taken at different locations, and the content of the X-type molecular sieve on the fiber surface was analyzed. The average content of molecular sieves on the fibers in the fifteen samples was 37 wt%, the standard deviation of the samples is 0.2 wt%, and the coefficient of variation is 0.5%, which indicates that the X-type molecular sieve is uniformly distributed on the fiber surface.

### Example 15

The preparation method of the X-type molecular sieve/polylactide-glycolide polymer fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with polylactide-glycolide polymer fiber, and the mass ratio of the polylactide-glycolide polymer fiber and the molecular sieve precursor solution is 1:20.
(ii) The polylactide-glycolide polymer fiber and the homogeneously-mixed molecular sieve precursor solution were heat-treated at 90 °C for 24 h to obtain a X-type molecular sieve/polylactide-glycolide polymer fiber composite material. The content of X-type molecular sieve was 20 wt%.

Fifteen samples of the prepared X-type molecular sieve/polylactide-glycolide polymer fiber composite material were randomly taken at different locations, and the content of the X-type molecular sieve on the fiber surface was analyzed. The average content of molecular sieves on the fibers in the fifteen samples was 20 wt%, the standard deviation of the samples is 0.04 wt%, and the coefficient of variation is 0.2%, which indicates that the X-type molecular sieve is uniformly distributed on the fiber surface.

### Example 16

The preparation method of the X-type molecular sieve/polyamide fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with polyamide fiber, and the mass ratio of the polyamide fiber and the molecular sieve precursor solution is 1:0.8.
(ii) The polyamide fiber and the homogeneously-mixed molecular sieve precursor solution were heat-treated at 90 °C for 24 h to obtain a X-type molecular sieve/polyamide fiber composite material. The content of X-type molecular sieve was 50 wt%.

Fifteen samples of the prepared X-type molecular sieve/polyamide fiber composite material were randomly taken at different locations, and the content of the X-type molecular sieve on the fiber surface was analyzed. The average content of molecular sieves on the fibers in the fifteen samples was 50 wt%, the standard deviation of the samples is 2 wt%, and the coefficient of variation is 4%, which indicates that the X-type molecular sieve is uniformly distributed on the fiber surface.

### Example 17

The preparation method of the X-type molecular sieve/rayon-polyester fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with rayon-polyester fiber, and the mass ratio of the rayon-polyester fiber and the molecular sieve precursor solution is 1:50.
(ii) The rayon-polyester fiber and the homogeneously-mixed molecular sieve precursor solution were heat-treated at 110 °C for 28 h to obtain a X-type molecular sieve/rayon-polyester fiber composite material. The content of X-type molecular sieve was 5 wt%.

Eight samples of the prepared X-type molecular sieve/rayon-polyester fiber composite material were randomly taken at different locations, and the content of the X-type molecular sieve on the fiber surface was analyzed. The average content of molecular sieves on the fibers in the eight samples was 5 wt%, the standard deviation of the samples is 0.05 wt%, and the coefficient of variation is 1%, which indicates that the X-type molecular sieve is uniformly distributed on the fiber surface.

### Example 18

The preparation method of the X-type molecular sieve/chitin fiber composite material of the present disclosure includes the following steps:
(i) A molecular sieve precursor solution was prepared, and a starting material was composed of 10Na₂O : Al₂O₃ : 9SiO₂ : 300H₂O in a molar ratio to synthesize a molecular sieve precursor solution. The molecular sieve precursor solution was mixed with chitin fiber, and the mass ratio of the chitin fiber and the molecular sieve precursor solution is 1:1.5.
(ii) The chitin fiber and the homogeneously-mixed molecular sieve precursor solution were heat-treated at 90 °C for 24 h to obtain a X-type molecular sieve/chitin fiber composite material. The content of X-type molecular sieve was 20 wt%.

Fifteen samples of the prepared X-type molecular sieve/chitin fiber composite material were randomly taken at different locations, and the content of the X-type molecular sieve on the fiber surface was analyzed. The average content of molecular sieves on the fibers in the fifteen samples was 20 wt%, the standard deviation of the samples is 2.5 wt%, and the coefficient of variation is 12.5%, which indicates that the X-type molecular sieve is uniformly distributed on the fiber surface.

### Example 19

The preparation method of the AlPO₄-5 molecular sieve/polyethylene fiber composite material of the present disclosure includes the following steps:
(i) A molecular sieve precursor solution was prepared, and a starting material was composed of Al₂O₃ : 1.3P₂O₅ : 1.3HF : 425H₂O : 6C₃H₇OH in a molar ratio to synthesize a molecular sieve precursor solution. The molecular sieve precursor solution was mixed with polyethylene fiber, and the mass ratio of the polyethylene fiber and the molecular sieve precursor solution is 1:20.
(ii) The polyethylene fiber and the homogeneously-mixed molecular sieve precursor solution were heat-treated at 180 °C for 6 h to obtain the AlPO₄-5 molecular sieve/polyethylene fiber composite material. The content of AlPO₄-5 molecular sieve was 18 wt%.

Fifteen samples of the prepared AlPO₄-5 molecular sieve/polyethylene fiber composite material were randomly taken at different locations, and the content of the AlPO₄-5 molecular sieve on the fiber surface was analyzed. The average content of molecular sieves on the fibers in the fifteen samples was 18 wt%, the standard deviation of the samples is 2.5 wt%, and the coefficient of variation is 13.9%, which indicates that the AlPO₄-5 molecular sieve is uniformly distributed on the fiber surface.

### Example 20

The preparation method of the AlPO₄-11 molecular sieve/polyvinyl chloride fiber composite material of the present disclosure includes the following steps:
(i) A molecular sieve precursor solution was prepared, and a starting material was composed of Al₂O₃ : 1.25P₂O₅ : 1.8HF : 156H₂O in a molar ratio to synthesize a molecular sieve precursor solution. The molecular sieve precursor solution was mixed with polyvinyl chloride fiber, and the mass ratio of the polyvinyl chloride fiber and the molecular sieve precursor solution is 1:0.5.
(ii) The polyvinyl chloride fiber and the homogeneously-mixed molecular sieve precursor solution were heat-treated at 145 °C for 18 h to obtain the AlPO₄-11 molecular sieve/polyvinyl chloride fiber composite material. The content of AlPO₄-11 molecular sieve was 28 wt%.

Fifteen samples of the prepared AlPO₄-11 molecular sieve/polyvinyl chloride fiber composite material were randomly taken at different locations, and the content of the AlPO₄-11 molecular sieve on the fiber surface was analyzed. The average content of molecular sieves on the fibers in the fifteen samples was 28 wt%, the standard deviation of the samples is 2 wt%, and the coefficient of variation is 7.1%, which indicates that the AlPO₄-11 molecular sieve is uniformly distributed on the fiber surface.

### Example 21

The preparation method of the SAPO-31 molecular sieve/polyacrylonitrile fiber composite material of the present disclosure includes the following steps:
(i) A molecular sieve precursor solution was prepared, and a starting material was composed of Al₂O₃ : P₂O₅ : 0.5SiO₂ : 60H₂O in a molar ratio to synthesize a molecular sieve precursor solution. The molecular sieve precursor solution was mixed with polyacrylonitrile fiber, and the mass ratio of the polyacrylonitrile fiber and the molecular sieve precursor solution is 1:1000.
(ii) The polyacrylonitrile fiber and the homogeneously-mixed molecular sieve precursor solution were heat-treated at 175 °C for 14.5 h to obtain a SAPO-31 molecular sieve/polyacrylonitrile fiber composite material. The content of SAPO-31 molecular sieve was 34 wt%.

Fifteen samples of the prepared SAPO-31 molecular sieve/polyacrylonitrile fiber composite material were randomly taken at different locations, and the content of the SAPO-31 molecular sieve on the fiber surface was analyzed. The average content of molecular sieves on the fibers in the fifteen samples was 34 wt%, the standard deviation of the samples is 5 wt%, and the coefficient of variation is 14.7%, which indicates that the SAPO-31 molecular sieve is uniformly distributed on the fiber surface.

### Example 22

The preparation method of the SAPO-34 molecular sieve/viscose fiber composite material of the present disclosure includes the following steps:
(i) A molecular sieve precursor solution was prepared, and a starting material was composed of Al₂O₃ : 1.06P₂O₅ : 1.08SiO₂ : 2.09morpholine : 60H₂O in a molar ratio to synthesize a molecular sieve precursor solution. The molecular sieve precursor solution was mixed with viscose fiber, and the mass ratio of the viscose fiber and the molecular sieve precursor solution is 1:20.
(ii) The viscose fiber and the homogeneously-mixed molecular sieve precursor solution were heat-treated at 175 °C for 14.5 h to obtain a SAPO-34 molecular sieve/viscose fiber composite material. The content of SAPO-34 molecular sieve was 1 wt%.

Fifteen samples of the prepared SAPO-34 molecular sieve/viscose fiber composite material were randomly taken at different locations, and the content of the SAPO-34 molecular sieve on the fiber surface was analyzed. The average content of molecular sieves on the fibers in the fifteen samples was 1 wt%, the standard deviation of the samples is 0.01 wt%, and the coefficient of variation is 1%, which indicates that the SAPO-34 molecular sieve is uniformly distributed on the fiber surface.

### Example 23

The preparation method of the SAPO-11 molecular sieve/chitin fiber composite material of the present disclosure includes the following steps:
(i) A molecular sieve precursor solution was prepared, and a starting material was composed of Al₂O₃ : P₂O₅ : 0.5SiO₂ : 60H₂O in a molar ratio to synthesize a molecular sieve precursor solution. The molecular sieve precursor solution was mixed with chitin fiber, and the mass ratio of the chitin fiber and the molecular sieve precursor solution is 1:1.5.
(ii) The chitin fiber and the homogeneously-mixed molecular sieve precursor solution were heat-treated at 175 °C for 48 h to obtain a SAPO-11 molecular sieve/chitin fiber composite material. The content of SAPO-11 molecular sieve was 35 wt%.

Fifteen samples of the prepared SAPO-11 molecular sieve/chitin fiber composite material were randomly taken at different locations, and the content of the SAPO-11 molecular sieve on the fiber surface was analyzed. The average content of molecular sieves on the fibers in the fifteen samples was 35 wt%, the standard deviation of the samples is 1.5 wt%, and the coefficient of variation is 5%, which indicates that the SAPO-11 molecular sieve is uniformly distributed on the fiber surface.

### Example 24

The preparation method of the BAC-1 molecular sieve/chitin fiber composite material of the present disclosure includes the following steps:
(i) A molecular sieve precursor solution was prepared, and a starting material was composed of 1.5B₂O₃ : 2.25Al₂O₃ : 2.5CaO : 200H₂O in a molar ratio to synthesize a molecular sieve precursor solution. The molecular sieve precursor solution was mixed with chitin fiber, and the mass ratio of the chitin fiber and the molecular sieve precursor solution is 1:100.
(ii) The chitin fiber and the homogeneously-mixed molecular sieve precursor solution were heat-treated at 200 °C for 72 h to obtain a BAC-1 molecular sieve/chitin fiber composite material. The content of BAC-1 molecular sieve was 0.5 wt%.

Fifteen samples of the prepared BAC-1 molecular sieve/chitin fiber composite material were randomly taken at different locations, and the content of the BAC-1 molecular sieve on the fiber surface was analyzed. The average content of molecular sieves on the fibers in the fifteen samples was 0.5 wt%, the standard deviation of the samples is 0.04 wt%, and the coefficient of variation is 8%, which indicates that the BAC-1 molecular sieve is uniformly distributed on the fiber surface.

### Example 25

The preparation method of the BAC-3 molecular sieve/chitin fiber composite material of the present disclosure includes the following steps:
(i) A molecular sieve precursor solution was prepared, and a starting material was composed of 3B₂O₃ : Al₂O₃ : 0.7Na₂O : 100H₂O in a molar ratio to synthesize a molecular sieve precursor solution. The molecular sieve precursor solution was mixed with chitin fiber, and the mass ratio of the chitin fiber and the molecular sieve precursor solution is 1:2.
(ii) The chitin fiber and the homogeneously-mixed molecular sieve precursor solution were heat-treated at 200 °C for 240 h to obtain a BAC-3 molecular sieve/chitin fiber composite material. The content of BAC-3 molecular sieve was 27 wt%.

Fifteen samples of the prepared BAC-3 molecular sieve/chitin fiber composite material were randomly taken at different locations, and the content of the BAC-3 molecular sieve on the fiber surface was analyzed. The average content of molecular sieves on the fibers in the fifteen samples was 27 wt%, the standard deviation of the samples is 0.08 wt%, and the coefficient of variation is 0.3%, which indicates that the BAC-3 molecular sieve is uniformly distributed on the fiber surface.

### Example 26

The preparation method of the BAC-10 molecular sieve/chitin fiber composite material of the present disclosure includes the following steps:
(i) A molecular sieve precursor solution was prepared, and a starting material was composed of 2.5B₂O₃ : 2Al₂O₃ :CaO : 200H₂O in a molar ratio to synthesize a molecular sieve precursor solution. The molecular sieve precursor solution was mixed with chitin fiber, and the mass ratio of the chitin fiber and the molecular sieve precursor solution is 1:20.
(ii) The chitin fiber and the homogeneously-mixed molecular sieve precursor solution were heat-treated at 160 °C for 72 h to obtain a BAC-10 molecular sieve/chitin fiber composite material. The content of BAC-10 molecular sieve was 21 wt%.

Fifteen samples of the prepared BAC-10 molecular sieve/chitin fiber composite material were randomly taken at different locations, and the content of the BAC-10 molecular sieve on the fiber surface was analyzed. The average content of molecular sieves on the fibers in the fifteen samples was 21 wt%, the standard deviation of the samples is 0.9 wt%, and the coefficient of variation is 4.2%, which indicates that the BAC-10 molecular sieve is uniformly distributed on the fiber surface.

### Comparative Examples 10 and 11

Commercially available granular molecular sieve materials (Quikclot) and Combat Gauze from Z-Medica Co., Ltd., were taken as Comparative Examples 10 and 11, respectively. The hemostatic function of the materials was evaluated using a rabbit femoral artery lethal model.

Among them, the commercial Combat Gauze is an inorganic hemostatic material (clay, kaolin) attached to the fiber surface. Observed from the scanning electron microscope, the inorganic hemostatic material is unevenly distributed on the fiber surface, and the material is not bonded to the fiber surface, and it is easy to fall off from the fiber surface (Figure 20). Clay retention rate on the gauze fiber is 10% or less under ultrasonic condition for 1 min; clay retention rate on the gauze fiber is 5% or less under ultrasonic condition for 5 min (Figure 21); clay retention rate on the gauze fiber is 5% or less under ultrasonic condition for 20 min. This defective structural form limits the hemostatic properties of the hemostatic product and risks causing sequelae or other side effects.

The certain size of the molecular sieve in the molecular sieve/fiber composite material can promote the uniform distribution of the molecular sieve on the fiber surface. The size of the molecular sieve and the average particle diameters of the inner and outer surface nanoparticles in the synthetic composite materials of Examples 1-26 are shown in Table 1, according to the observation of the scanning electron microscope. In order to evaluate the binding strength between the molecular sieve and the fiber, the synthetic molecular sieve/fiber composite materials of Examples 1-26 were ultrasonicated in deionized water for 20, 40, 60, and 80 minutes, respectively. After ultrasonic testing, the retention rates of the molecular sieve on the fibers are shown in Table 2. In order to show that the molecular sieve in the molecular sieve/fiber composite material of the present disclosure maintains a good structure and performance on the fiber, after testing, the effective specific surface area and ion exchange capacity of the molecular sieve of Example 1-26 are shown in Table 3. In order to illustrate the superior hemostatic properties of composite material, a rabbit femoral artery lethal model was used to evaluate the hemostatic function of molecular sieve/fiber composite materials of Examples 1-26 and hemostatic materials of Comparative Examples. After observing and testing, statistical data of hemostatic performance are shown in Table 4.

**Table 1. The particle size of molecular sieve of the molecular sieve/fiber composite materials and the average particle size of the nanoparticles on the inner and outer surfaces**

| Serial number | Material | Molecular sieve D90/µm | Molecular sieve D50/µm | Average particle size of the nanoparticles on the outer surfaces /nm | Average particle size of the nanoparticles on the inner surfaces /nm |
|---|---|---|---|---|---|
| Example 1 | Y-type molecular sieve/cotton fiber composite material | 25 | 5 | 148 | 61 |
| Example 2 | Chabazite/cotton fiber composite material | 4 | 2 | 200 | 31 |
| Example 3 | X-type molecular sieve/silk fiber composite material | 20 | 10 | 256 | 50 |
| Example 4 | A-type molecular sieve/polyester fiber composite material | 50 | 30 | 141 | 12 |
| Example 5 | ZSM-5 molecular sieve/polypropylene fiber composite material | 30 | 15 | 190 | 11 |
| Example 6 | β-molecular sieve/rayon fiber composite material | 6 | 4 | 110 | 33 |
| Example 7 | Mordenite/acetate fiber composite material | 7 | 3 | 109 | 23 |
| Example 8 | L-type molecular sieve/carboxymethyl cellulose composite material | 8 | 5.5 | 300 | 22 |
| Example 9 | P-type molecular sieve/bamboo fiber composite material | 10 | 8 | 240 | 60 |
| Example 10 | Merlinoite/linen fiber composite material | 5 | 1 | 200 | 12 |
| Example 11 | X-type molecular sieve/wool composite material | 10 | 5 | 240 | 4 |
| Example 12 | X-type molecular sieve/wood fiber composite material | 0.1 | 0.05 | 3 | 2 |
| Example 13 | X-type molecular sieve/lactide polymer fiber composite material | 0.01 | 0.005 | 3 | 2 |
| Example 14 | X-type molecular sieve/glycolide polymer fiber composite material | 0.5 | 0.25 | 10 | 4 |
| Example 15 | X-type molecular sieve/polylactide-glycolide polymer fiber composite material | 1 | 0.5 | 30 | 20 |
| Example 17 | X-type molecular sieve/rayon-polyester fiber composite material | 20 | 13 | 195 | 68 |
| Example 18 | X-type molecular sieve/chitin fiber composite material | 20 | 10 | 150 | 100 |
| Example 19 | AlPO₄-5 molecular sieve/polyethylene fiber composite material | 7.5 | 5.5 | 500 | 22 |
| Example 20 | AlPO₄-11 molecular sieve/polyvinyl chloride fiber composite material | 5 | 4 | 200 | 2 |
| Example 21 | SAPO-31 molecular sieve/polyacrylonitrile fiber composite material | 3 | 3 | 109 | 25 |
| Example 22 | SAPO-34 molecular sieve/viscose fiber composite material | 5 | 4 | 110 | 33 |
| Example 23 | SAPO-11 molecular sieve/chitin fiber composite material | 8 | 5 | 211 | 10 |
| Example 24 | BAC-1 molecular sieve/chitin fiber composite material | 12 | 10 | 256 | 51 |
| Example 25 | BAC-3 molecular sieve/chitin fiber composite material | 15 | 8 | 500 | 32 |
| Example 26 | BAC-10 molecular sieve/chitin fiber composite material | 10 | 8 | 50 | 4 |

**Table 2. The binding strength of molecular sieve and fiber of molecular sieve/fiber composite materials**

| Serial number | Material | Retention rate of molecular sieves on fibers under ultrasonic condition for 20 min | Retention rate of molecular sieves on fibers under ultrasonic condition for 40 min | Retention rate of molecular sieves on fibers under ultrasonic condition for 60 min | Retention rate of molecular sieves on fibers under ultrasonic condition for 80 min |
|---|---|---|---|---|---|
| Example 1 | Y-type molecular sieve/cotton fiber composite material | 100% | 100% | 100% | 100% |
| Example 2 | Chabazite/cotton fiber composite material | 100% | 100% | 100% | 100% |
| Example 3 | X-type molecular sieve/silk fiber composite material | 95% | 95% | 95% | 95% |
| Example 4 | A-type molecular sieve/polyester fiber composite material | 100% | 100% | 100% | 100% |
| Example 5 | ZSM-5 molecular sieve/polypropylene fiber composite material | 98% | 98% | 98% | 98% |
| Example 6 | β-molecular sieve/rayon fiber composite material | 100% | 100% | 100% | 100% |
| Example 7 | Mordenite/acetate fiber composite material | 91% | 91% | 91% | 91% |
| Example 8 | L-type molecular sieve/carboxymethyl cellulose composite material | 99% | 99% | 99% | 99% |
| Example 9 | P-type molecular sieve/bamboo fiber composite material | 100% | 100% | 100% | 100% |
| Example 10 | Merlinoite/linen fiber composite material | 100% | 100% | 100% | 100% |
| Example 11 | X-type molecular sieve/wool composite material | 90% | 90% | 90% | 90% |
| Example 12 | X-type molecular sieve/wood fiber composite material | 100% | 100% | 100% | 100% |
| Example 13 | X-type molecular sieve/lactide polymer fiber composite material | 100% | 100% | 100% | 100% |
| Example 14 | X-type molecular sieve/glycolide polymer fiber composite material | 100% | 100% | 100% | 100% |
| Example 15 | X-type molecular sieve/polylactide-glycoli de polymer fiber composite material | 100% | 100% | 100% | 100% |
| Example 16 | X-type molecular sieve/polyamide fiber composite material | 94% | 94% | 94% | 94% |
| Example 17 | X-type molecular sieve/rayon-polyester fiber composite material | 96% | 96% | 96% | 96% |
| Example 18 | X-type molecular sieve/chitin fiber composite material | 91% | 91% | 91% | 91% |
| Example 19 | AlPO₄-5 molecular sieve/polyethylene fiber composite material | 100% | 100% | 100% | 100% |
| Example 20 | AlPO₄-11 molecular sieve/polyvinyl chloride fiber composite material | 100% | 100% | 100% | 100% |
| Example 21 | SAPO-31 molecular sieve/polyacrylonitrile fiber composite material | 90% | 90% | 90% | 90% |
| Example 22 | SAPO-34 molecular sieve/viscose fiber composite material | 100% | 100% | 100% | 100% |
| Example 23 | SAPO-11 molecular sieve/chitin fiber composite material | 100% | 100% | 100% | 100% |
| Example 24 | sieve/chitin fiber composite material | 100% | 100% | 100% | 100% |
| Example 25 | BAC-3 molecular sieve/chitin fiber composite material | 100% | 100% | 100% | 100% |
| Example 26 | BAC-10 molecular sieve/chitin fiber composite material | 99% | 99% | 99% | 99% |

**Table 3. Effective specific surface area and ion exchange capacity of molecular sieves of different molecular sieve/fiber composite materials**

| Serial number | Material | Effective specific surface area of molecular sieves/(m²g⁻¹) | Degree of calcium ion exchange | Degree of magnesium ion exchange | Degree of Strontium ion exchange |
|---|---|---|---|---|---|
| Example 1 | Y-type molecular sieve/cotton fiber composite material | 490 | 99.9% | 97% | 90% |
| Example 2 | Chabazite/cotton fiber composite material | 853 | 90.2% | 92% | 80% |
| Example 3 | X-type molecular sieve/silk fiber composite material | 741 | 91% | 81% | 80% |
| Example 4 | A-type molecular sieve/polyester fiber composite material | 502 | 85% | 77% | 70% |
| Example 5 | ZSM-5 molecular sieve/polypropylene fiber composite material | 426 | 80% | 77% | 70% |
| Example 6 | β-molecular sieve/rayon fiber composite material | 763 | 95% | 87% | 85% |
| Example 7 | Mordenite/acetate fiber composite material | 412 | 95% | 87% | 85% |
| Example 8 | L-type molecular sieve/carboxymethyl cellulose composite material | 858 | 85% | 81% | 80% |
| Example 9 | P-type molecular sieve/bamboo fiber composite material | 751 | 91% | 90% | 85% |
| Example 10 | Merlinoite/linen fiber composite material | 510 | 98.5% | 97% | 90% |
| Example 11 | X-type molecular sieve/wool composite material | 494 | 98% | 97% | 91% |
| Example 12 | X-type molecular sieve/wood fiber composite material | 492 | 99% | 97% | 93% |
| Example 13 | X-type molecular sieve/lactide polymer fiber composite material | 496 | 98.9% | 97% | 90% |
| Example 14 | X-type molecular sieve/glycolide polymer fiber composite material | 480 | 97% | 97% | 91% |
| Example 15 | X-type molecular sieve/polylactide-glycolide polymer fiber composite material | 499 | 99.7% | 95% | 87% |
| Example 16 | X-type molecular sieve/polyamide fiber composite material | 495 | 95% | 94% | 90% |
| Example 17 | X-type molecular sieve/rayon-polyester fiber composite material | 846 | 91.2% | 90% | 83% |
| Example 18 | X-type molecular sieve/chitin fiber composite material | 751 | 91% | 90% | 85% |
| Example 19 | AlPO₄-5 molecular sieve/polyethylene fiber composite material | 426 | - | - | - |
| Example 20 | AlPO₄-11 molecular sieve/polyvinyl chloride fiber composite material | 763 | - | - | - |
| Example 21 | SAPO-31 molecular sieve/polyacrylonitrile fiber composite material | 412 | - | - | - |
| Example 22 | SAPO-34 molecular sieve/viscose fiber composite material | 858 | - | - | - |
| Example 23 | SAPO-11 molecular sieve/chitin fiber composite material | 510 | - | - | - |
| Example 24 | BAC-1 molecular sieve/chitin fiber composite material | 494 | - | - | - |
| Example 25 | BAC-3 molecular sieve/chitin fiber composite material | 492 | - | - | - |
| Example 26 | BAC-10 molecular sieve/chitin fiber composite material | 496 | - | - | - |

**Table 4. Hemostatic function of different hemostatic materials**

| Serial number | Hemostatic material | Hemo static time | Rising temperature of wound (°C) | Blood loss (g) | Ease of use | Debridement effect | Wound condition | Survival rate |
|---|---|---|---|---|---|---|---|---|
| Example 1 | Y-type molecular sieve/cotton fiber composite material | 2 min | No | 4 ± 0.5 | Tailored for wound size and practical needs | Easy to remove, no other removal required | Dry and well healed | 100% |
| Example 2 | Chabazite/cott on fiber composite material | 1.8 min | No | 3 ± 0.5 | Tailore d for wound size and practical needs | Easy to remove, no other removal required | Dry and well healed | 100% |
| Example 3 | X-type molecular sieve/silk fiber composite material | 1.8 min | No | 3 ± 0.4 | Tailored for wound size and practical needs | Easy to remove, no other removal required | Dry and well healed | 100% |
| Example 4 | A-type molecular sieve/polyester fiber composite material | 2 min | No | 3 ± 0.8 | Tailored for wound size and practical needs | Easy to remove, no other removal required | Dry and well healed | 100% |
| Example 5 | ZSM-5 molecular sieve/polyprop ylene fiber composite material | 2.5 min | No | 4 ± 0.8 | Tailored for wound size and practical needs | Easy to remove, no other removal required | Dry and well healed | 100% |
| Example 6 | β-molecular sieve/rayon fiber composite material | 2 min | No | 3 ± 0.8 | Tailored for wound size and practical needs | Easy to remove, no other removal required | Dry and well healed | 100% |
| Example 7 | Mordenite/acet ate fiber composite material | 2.1 min | No | 3.5 ± 0.4 | Tailored for wound size and practical needs | Easy to remove, no other removal required | Dry and well healed | 100% |
| Example 8 | L-type molecular sieve/carboxy methyl cellulose composite material | 2.7 min | No | 4.2 ± 0.4 | Tailored for wound size and practical needs | Easy to remove, no other removal required | Dry and well healed | 100% |
| Example 9 | P-type molecular sieve/bamboo fiber composite material | 2.4 min | No | 4.3 ± 0.7 | Tailored for wound size and practical needs | Easy to remove, no other removal required | Dry and well healed | 100% |
| Example 10 | Merlinoite/line n fiber composite material | 2.4 min | No | 4.3 ± 0.7 | Tailored for wound size and practical needs | Easy to remove, no other removal required | Dry and well healed | 100% |
| Example 11 | X-type molecular sieve/wool composite material | 2 min | No | 4 ± 0.5 | Tailored for wound size and practical needs | Easy to remove, no other removal required | Dry and well healed | 100% |
| Example 12 | X-type molecular sieve/wood fiber composite material | 2.1 min | No | 4 ± 0.5 | Tailored for wound size and practical needs | Easy to remove, no other removal required | Dry and well healed | 100% |
| Example 13 | X-type molecular sieve/l actide polymer fiber composite material | 2.4 min | No | 4.3 ± 0.3 | Tailored for wound size and practical needs | Easy to remove, no other removal required | Dry and well healed | 100% |
| Example 14 | X-type molecular sieve/glycolide polymer fiber composite material | 2.1 min | No | 3.5 ± 0.5 | Tailored for wound size and practical needs | Easy to remove, no other removal required | Dry and well healed | 100% |
| Example 15 | X-type molecular sieve/polylacti de-glycolide polymer fiber composite material | 2.3 min | No | 3.8± 0.5 | Tailored for wound size and practical needs | Easy to remove, no other removal required | Dry and well healed | 100% |
| Example 16 | X-type molecular sieve/polyamid e fiber composite material | 2.5 min | No | 4.2 ± 0.5 | Tailored for wound size and practical needs | Easy to remove, no other removal required | Dry and well healed | 100% |
| Example 17 | X-type molecular sieve/rayon-po lyester fiber composite material | 2.4 min | No | 4.4 ± 0.5 | Tailored for wound size and practical needs | Easy to remove, no other removal required | Dry and well healed | 100% |
| Example 18 | X-type molecular sieve/chitin fiber composite material | 2 min | No | 3 ± 0.8 | Tailored for wound size and practical needs | Easy to remove, no other removal required | Dry and well healed | 100% |
| Example 19 | AlPO₄-5 molecular sieve/polyethyl ene fiber composite material | 2 min | No | 3 ± 0.8 | Tailored for wound size and practical needs | Easy to remove, no other removal required | Dry and well healed | 100% |
| Example 20 | AlPO₄-11 molecular sieve/polyvinyl chloride fiber composite material | 2.1 min | No | 3.5 ± 0.4 | Tailored for wound size and practical needs | Easy to remove, no other removal required | Dry and well healed | 100% |
| Example 21 | SAPO-31 molecular sieve/polyacryl onitrile fiber composite material | 2.1 min | No | 3.2 ± 0.4 | Tailored for wound size and practical needs | Easy to remove, no other removal required | Dry and well healed | 100% |
| Example 22 | SAPO-34 molecular sieve/viscose fiber composite material | 2.4 min | No | 4 ± 0.7 | Tailored for wound size and practical needs | Easy to remove, no other removal required | Dry and well healed | 100% |
| Example 23 | SAPO-11 molecular sieve/chitin fiber composite material | 2.5 min | No | 4.2 ± 0.5 | Tailored for wound size and practical needs | Easy to remove, no other removal required | Dry and well healed | 100% |
| Example 24 | BAC-1 molecular sieve/chitin fiber composite material | 2.4 min | No | 4.4 ± 0.5 | Tailored for wound size and practical needs | Easy to remove, no other removal required | Dry and well healed | 100% |
| Example 25 | BAC-3 molecular sieve/chitin fiber composite material | 2 min | No | 3 ± 0.8 | Tailored for wound size and practical needs | Easy to remove, no other removal required | Dry and well healed | 100% |
| Example 26 | BAC-10 molecular sieve/chitin fiber composite material | 2.4 min | No | 3.4 ± 0.1 | Tailored for wound size and practical needs | Easy to remove, no other removal required | Dry and well healed | 100% |
| Comparative Example 2 | Y-type molecular sieve/cotton fiber complex material (impregnation method) | 5.4 min | 2 ± 1 | 7.4 ± 0.1 | Tailored for wound size and practical needs | Part of the molecular sieves fall from the fiber and stick to the wound, making them difficult to remove | A large blood clot forms on the surface of the wound, which is generally healed | 60% |
| Comparative Example 3 | Y-type molecular sieve/cotton fiber complex material (spray method) | 5.2 min | 3 ± 1 | 7.6 ± 0.1 | Tailored for wound size and practical needs | Part of the molecular sieves fall from the fiber and stick to the wound, making them difficult to remove | A large blood clot forms on the surface of the wound, which is generally healed | 55% |
| Comparative Example 4 | Y-type molecular sieve/cotton fiber complex material (including adhesive 1) | 6.2 min | 7 ± 1 | 5.5 ± 0.2 | Tailored for wound size and practical needs | Part of the molecular sieves fall from the fiber and stick to the wound, making them difficult to remove | A large blood clot forms on the surface of the wound, which is generally healed | 65% |
| Comparative Example 5 | Y-type molecular sieve/cotton fiber complex material (including adhesive 2) | 5.4 min | 4 ± 1 | 8.5 ± 0.2 | Tailored for wound size and practical needs | Part of the molecular sieves fall from the fiber and stick to the wound making them difficult to remove | A large blood clot forms on the surface of the wound, which is generally healed | 45% |
| Comparative Example 6 | Y-type molecular sieve/cotton fiber complex material (including adhesive 3) | 6 min | 2 ± 1 | 7.5 ± 0.1 | Tailored for wound size and practical needs | Part of the molecular sieves fall from the fiber and stick to the wound, making them difficult to remove | A large blood clot forms on the surface of the wound, which is generally healed | 40% |
| Comparative Example 7 | Y-type molecular sieve/cotton fiber complex material (pretreatment of fiber) | 6.5 min | 2 ± 1 | 7.1 ± 0.1 | hard and brittle, and does not make good contact with the wound | Part of the molecular sieves fall from the fiber and stick to the wound, making them difficult to remove | A large blood clot forms on the surface of the wound, which is generally healed | 45% |
| Comparative Example 8 | Y-type molecular sieve/spandex fiber complex material (blend spinning) | 5.4 min | No | 9.1 ± 0.1 | Tailored for wound size and practical needs | Easy to remove, no other removal required | A large blood clot forms on the surface of the wound, which is generally healed | 40% |
| Comparative Example 10 | Quikclot molecular sieve granule | 3 min | 10 ± 2 | 6.5 ± 0.9 | Difficult to adjust dosage | The granules need to be washed several times with physiological saline, and can plug in blood vessels | The wound with slight burns was washed by physiological saline, and it was easy to rebleed. | 50% |
| Comparative Example 11 | Combat Gauze (Clay/fiber complex material) | 7.5 min | No | 12.7 ± 0.8 | Tailored for wound size and practical needs | Part of the clay falls off the fibers. Due to the large amount of bleeding, a large area of blood clot is formed on the wound surface, which adheres to the wound surface and is easy to rebleed when cleared. | A large blood clot forms on the wound surface, making it difficult to observe the actual blood vessel healing | 40% |

The above results show that: Examples 1-26 list the synthesis of molecular sieves/fiber composite material with different molecular sieves and different fibers, and the molecular sieves of synthesized molecular sieves/fiber composite materials are uniformly distributed on the fiber surface of the fibers in a suitable size, so that the overall performance of the composite material remains consistent. And the inner surface of the molecular sieves of the molecular sieve/fiber composite materials of Examples 1-26 in contact with the fibers is a rough planar surface. Ultrasound the molecular sieve/fiber composite materials in deionized water for ≥ 20 min, and use a thermogravimetric analyzer to analyze the content of molecular sieves on the fiber surface. The retention rate of molecular sieves is ≥ 90%, indicating that a growth-matched coupling is formed between the molecular sieve and the fiber. The molecular sieve is firmly bonded to the fiber. The adhesive content of the contact surface between the molecular sieve and the fiber is zero in Examples 1-26 of the present disclosure, and the degree of calcium ion exchange of the molecular sieve is ≥ 90%, the degree of magnesium ion exchange is ≥ 75%, and the degree of strontium ion exchange is ≥ 70%. It overcomes the defects of high synthetic cost, low effective surface area of molecular sieve, and clogging of molecular sieve channels, which exist on the composite materials through the adhesive. In addition, the molecular sieves are independently dispersed on the fiber surface to promote the uniform distribution of the molecular sieves on the fiber surface. The inner surface of the molecular sieves is composed of nanoparticles without corner angles. The nanoparticles without corner angles make the inner surface of the molecular sieves match the fiber surface better, which is beneficial to the combination of the molecular sieve and the fiber. The method for synthesizing molecular sieve/fiber composite materials does not include a templating agent, and the molecular sieve is a mesoporous molecular sieve. The method has the characteristics of low cost, simple process, and environmental friendliness.

The present disclosure also provides a hemostatic material of molecular sieve/fiber composite material. Although the hemostatic material of molecular sieve/fiber composite material has a reduced amount of molecular sieve compared with the molecular sieve granules, the hemostatic effect of the molecular sieve/fiber composite material is better than the commercial molecular sieve granules (Quikclot), which further solves the problem of water absorption and heat release. The molecular sieves of the present disclosure are uniformly distributed on the fiber surface with a certain size, and a growth-matched coupling is formed between the molecular sieve and the fiber. The molecular sieve has a strong binding strength with the fiber. The molecular sieve has a high effective specific surface area and substance exchange capacity on the fiber surface. The hemostatic effect of hemostatic materials of the present disclosure is superior to that of composite materials with weak binding strength between molecular sieves and fiber or low effective specific surface area or low material exchange capacity in the prior art. The molecular sieve/fiber composite materials have a short hemostatic time, low blood loss, and high survival rate in the rabbit femoral artery lethal model, and the molecular sieve/fiber composite materials are safe during hemostatic process. In addition, the molecular sieve/fiber composite materials also have the following advantages: (i) the wound surface after hemostasis is easy to clean up and convenient for post-processing by professionals; (ii) the molecular sieve/fiber composite materials can be tailored for wound size and practical needs; (iii) the wound after hemostasis is dry and heals well after treated with the molecular sieve/fiber composite materials.

The above embodiments are only used to illustrate the present disclosure and are not used to limit the scope of the present disclosure. In addition, it should be understood that after reading the teaching of the present disclosure, those skilled in the art can make various changes or modifications to the present disclosure, and these equivalent also fall within the scope defined by the appended claims of the present application.

## Claims

1. A molecular sieve/fiber composite material, **characterized in that** the molecular sieve/fiber composite material comprises molecular sieves and a fiber, the molecular sieves are distributed on a fiber surface and directly contact the fiber surface; a particle diameter D90 of the molecular sieves is 0.01 to 50 µm, a particle size D50 of the molecular sieves is 0.005 to 30 µm;
the molecular sieves are distributed uniformly on the fiber surface of the fiber; a detection method of uniform distribution of molecular sieves on the fiber surface is: randomly taking n samples of the molecular sieve/fiber composite material at different locations, and analyzing the content of the molecular sieve on the fiber surface, where n is a positive integer greater than or equal to 8, a coefficient of variation of the content of the molecular sieves in the n samples is ≤ 15%.

2. The molecular sieve/fiber composite material of claim 1, **characterized in that** the particle diameter D90 of the molecular sieve is 0.1 to 30 µm, and the particle diameter D50 of the molecular sieve is 0.05 to 15 µm; preferably, the particle diameter D90 of the molecular sieve is 0.5 to 20 µm, and the particle diameter D50 of the molecular sieve is 0.25 to 10 µm; preferably, the particle diameter D90 of the molecular sieve is 1 to 15 µm, and the particle diameter D50 of the molecular sieve is 0.5 to 8 µm; more preferably, the particle diameter D90 of the molecular sieve is 5 to 10 µm, and the particle diameter D50 of the molecular sieve is 2.5 to 5 µm.

3. The molecular sieve/fiber composite material of claim 1, **characterized in that** the coefficient of variation of the content of the molecular sieves is ≤ 10%; preferably, the coefficient of variation of the content of the molecular sieves is ≤ 5%; preferably, the coefficient of variation of the content of the molecular sieves is ≤ 2%; preferably, the coefficient of variation of the content of the molecular sieves is ≤ 1%; preferably, the coefficient of variation of the content of the molecular sieves is ≤ 0.5%; preferably, the coefficient of variation of the content of the molecular sieves is ≤ 0.2%.

4. The molecular sieve/fiber composite material of claim 1, **characterized in that** the adhesive content of the contact surface between the molecular sieve and the fiber is zero.

5. The molecular sieve/fiber composite material of claim 1, **characterized in that** the surface of molecular sieve contacted with the fiber is defined as an inner surface, and the inner surface is a rough planar surface; growth-matched coupling is formed between the molecular sieve and the fiber on the inner surface of the molecular sieve; the surface of molecular sieve uncontacted with the fiber is defined as an outer surface, and the outer surface is a non-planar surface.

6. The molecular sieve/fiber composite material of claim 5, **characterized in that** a detection method for forming a growth-matched coupling is performed in conditions as follows: the retention rate of the molecular sieve on the fiber is greater than or equal to 90% under ultrasonic condition for 20 minutes or more.

7. The molecular sieve/fiber composite material of claim 5, **characterized in that** a detection method for forming a growth-matched coupling is performed in conditions as follows: the retention rate of the molecular sieve on the fiber is greater than or equal to 90% under ultrasonic condition for 40 minutes or more.

8. The molecular sieve/fiber composite material of claim 5, **characterized in that** a detection method for forming a growth-matched coupling is performed in conditions as follows: the retention rate of the molecular sieve on the fiber is greater than or equal to 90% under ultrasonic condition for 60 minutes or more.

9. The molecular sieve/fiber composite material according to any one of claims 6-8, **characterized in that** the retention rate is ≥ 95%; more preferably, the retention rate is 100%.

10. The molecular sieve/fiber composite material of claim 5, **characterized in that** the inner surface and outer surface are composed of molecular sieve nanoparticles.

11. The molecular sieve/fiber composite material of claim 10, **characterized in that** the average size of the molecular sieve nanoparticles of the outer surface is larger than the average size of the molecular sieve nanoparticles of the inner surface.

12. The molecular sieve/fiber composite material according to any one of claims 1-11, **characterized in that** the molecular sieves are independently dispersed on the fiber surface.

13. The molecular sieve/fiber composite material of claim 12, **characterized in that** the independent dispersion means that the molecular sieve microparticles are dispersed on the fiber surface with a gap between each other.

14. The molecular sieve/fiber composite material of claim 12, **characterized in that** the independent dispersion means the minimum distance between the molecular sieve microparticle and the nearest molecular sieve microparticle is greater than or equal to one half of the sum of the particle sizes of the two molecular sieve microparticles.

15. The molecular sieve/fiber composite material of claim 10, **characterized in that** the molecular sieve nanoparticles of outer surface are particles with corner angles.

16. The molecular sieve/fiber composite material of claim 10, **characterized in that** the molecular sieve nanoparticles of inner surface are particles without corner angles.

17. The molecular sieve/fiber composite material of claim 10, **characterized in that** the average size of the molecular sieve nanoparticles of the inner surface is 2 to 100 nm; preferably, the average size of the molecular sieve nanoparticles of inner surface is 10 to 60 nm.

18. The molecular sieve/fiber composite material of claim 10, **characterized in that** the average size of the molecular sieve nanoparticles of the outer surface is 50 to 500 nm; preferably, the average size of the molecular sieve nanoparticles of outer surface is 100 to 300 nm.

19. The molecular sieve/fiber composite material of claim 5, **characterized in that** the non-planar surface is composed of any one or combination of non-planar curves or non-planar lines.

20. The molecular sieve/fiber composite material of claim 19, **characterized in that** the non-planar surface is a spherical surface.

21. The molecular sieve/fiber composite material of claim 1, **characterized in that** the molecular sieve is a mesoporous molecular sieve.

22. The molecular sieve/fiber composite material according to any one of claims 1-21, the content of the molecular sieve accounts for 0.05 to 80 wt% of the molecular sieve/fiber composite material; preferably, the content of the molecular sieve accounts for 1 to 50 wt% of the molecular sieve/fiber composite material; preferably, the content of the molecular sieve accounts for 5 to 35 wt% of the molecular sieve/fiber composite material; preferably, the content of the molecular sieve accounts for 10 to 25 wt% of the molecular sieve/fiber composite material; more preferably, the content of the molecular sieve accounts for 15 to 20 wt% of the molecular sieve/fiber composite material.

23. The molecular sieve/fiber composite material of claim 1, **characterized in that** the molecular sieve is selected from the group consisting of aluminosilicate molecular sieve, phosphate molecular sieve, borate molecular sieve, heteroatom molecular sieve, and combination thereof.

24. The molecular sieve/fiber composite material of claim 23, **characterized in that** the aluminosilicate molecular sieve is selected from the group consisting of X-type molecular sieve, Y-type molecular sieve, A-type molecular sieve, ZSM-5 molecular sieve, chabazite, β-molecular sieve, mordenite, L-type molecular sieve, P-type molecular sieve, merlinoite, and combination thereof.

25. The molecular sieve/fiber composite material of claim 23, **characterized in that** the phosphate molecular sieve is selected from the group consisting of AlPO₄-5 molecular sieve, AlPO₄-11 molecular sieve, SAPO-31 molecular sieve, SAPO-34 molecular sieve, SAPO-11, and combination thereof.

26. The molecular sieve/fiber composite material of claim 23, **characterized in that** the borate molecular sieve is selected from the group consisting of BAC-1 molecular sieve, BAC-3 molecular sieve, BAC-10 molecular sieve, and combination thereof.

27. The molecular sieve/fiber composite material of claim 23, **characterized in that** the heteroatom molecular sieve is that a heteroatom element replaces part of silicon, aluminum, and phosphorus of the molecular sieve framework to form a molecular sieve containing other elements.

28. The molecular sieve/fiber composite material of claim 27, **characterized in that** the heteroatom element is selected from the group consisting of transition metal elements in the fourth, fifth, sixth periods, and combination thereof.

29. The molecular sieve/fiber composite material according to any one of claims 1-28, **characterized in that** the molecular sieve is a molecular sieve after metal ion exchange.

30. The molecular sieve/fiber composite material of claim 29, **characterized in that** the metal ion is selected from the group consisting of strontium ion, calcium ion, magnesium ion, silver ion, zinc ion, barium ion, potassium ion, ammonium ion, copper ion, and combination thereof.

31. The molecular sieve/fiber composite material according to any one of claims 1-30, **characterized in that** the fiber is a polymer containing hydroxyl groups in a repeating unit.

32. The molecular sieve/fiber composite material according to any one of claims 1-30, **characterized in that** the fiber is selected from the group consisting of silk fiber, chitin fiber, rayon fiber, acetate fiber, carboxymethyl cellulose, bamboo fiber, cotton fiber, linen fiber, wool, wood fiber, lactide polymer fiber, glycolide polymer fiber, polyester fiber, polyamide fiber, polypropylene fiber, polyethylene fiber, polyvinyl chloride fiber, polyacrylonitrile fiber, viscose fiber, and combination thereof.

33. A preparation method for a molecular sieve/fiber composite material of claim 1, **characterized in that** the preparation method is an in-situ growth method, and the in-situ growth method comprises the following steps:
(i) preparing a molecular sieve precursor solution and mixing the molecular sieve precursor solution with a fiber;
(ii) processing the mixture of the fiber and the molecular sieve precursor solution obtained in step (i) with heat treatment to obtain the molecular sieve/fiber composite material.

34. The preparation method of claim 33, **characterized in that** the molecular sieve precursor solution does not include a templating agent.

35. The preparation method of claim 33, **characterized in that** in the step (ii), the temperature of the heat treatment is 60 to 220 °C, and the time of heat treatment is 4 to 240 h.

36. The preparation method of claim 33, **characterized in that** in the step (i), mass ratio of the fiber to the molecular sieve precursor solution is 1:0.5 to 1:1000; preferably, in the step (i), mass ratio of the fiber to the molecular sieve precursor solution is 1:0.8 to 1:100; preferably, in the step (i), mass ratio of the fiber to the molecular sieve precursor solution is 1:1 to 1:50; preferably, in the step (i), mass ratio of the fiber to the molecular sieve precursor solution is 1:1.5 to 1:20; preferably, in the step (i), mass ratio of the fiber to the molecular sieve precursor solution in is 1:2 to 1:10; more preferably, in the step (i), mass ratio of the fiber to the molecular sieve precursor solution is 1:2 to 1:5.

37. The preparation method of claim 33, **characterized in that** the molecular sieve is a mesoporous molecular sieve.

38. The preparation method of claim 33, **characterized in that** the particle diameter D90 of the molecular sieve is 0.1 to 30 µm, and the particle diameter D50 of the molecular sieve is 0.05 to 15 µm; preferably, the particle diameter D90 of the molecular sieve is 0.5 to 20 µm, and the particle diameter D50 of the molecular sieve is 0.25 to 10 µm; preferably, the particle diameter D90 of the molecular sieve is 1 to 15 µm, and the particle diameter D50 of the molecular sieve is 0.5 to 8 µm; more preferably, the particle diameter D90 of the molecular sieve is 5 to 10 µm, and the particle diameter D50 of the molecular sieve is 2.5 to 5 µm.

39. The preparation method of claim 33, **characterized in that** the coefficient of variation of the content of the molecular sieves is ≤ 10%; preferably, the coefficient of variation of the content of the molecular sieves is ≤ 5%; preferably, the coefficient of variation of the content of the molecular sieves is ≤ 2%; preferably, the coefficient of variation of the content of the molecular sieves is ≤ 1%; preferably, the coefficient of variation of the content of the molecular sieves is ≤ 0.5%; preferably, the coefficient of variation of the content of the molecular sieves is ≤ 0.2%.

40. The preparation method of claim 33, **characterized in that** the adhesive content of the contact surface between the molecular sieve and the fiber is zero.

41. The preparation method of claim 33, **characterized in that** the surface of molecular sieve contacted with the fiber is defined as an inner surface, and the inner surface is a rough planar surface; growth-matched coupling is formed between the molecular sieve and the fiber on the inner surface of the molecular sieve; the surface of molecular sieve uncontacted with the fiber is defined as an outer surface, and the outer surface is a non-planar surface.

42. The preparation method of claim 41, **characterized in that** a detection method for forming a growth-matched coupling is performed in conditions as follows: the retention rate of the molecular sieve on the fiber is greater than or equal to 90% under ultrasonic condition for 20 minutes or more.

43. The preparation method of claim 41, **characterized in that** a detection method for forming a growth-matched coupling is performed in conditions as follows: the retention rate of the molecular sieve on the fiber is greater than or equal to 90% under ultrasonic condition for 40 minutes or more.

44. The preparation method of claim 41, **characterized in that** a detection method for forming a growth-matched coupling is performed in conditions as follows: the retention rate of the molecular sieve on the fiber is greater than or equal to 90% under ultrasonic condition for 60 minutes or more.

45. The preparation method according to any one of claims 42-44, **characterized in that** the retention rate is ≥ 95%; more preferably, the retention rate is 100%.

46. The preparation method of claim 41, **characterized in that** the inner surface and outer surface are composed of molecular sieve nanoparticles.

47. The preparation method of claim 46, **characterized in that** the average size of the molecular sieve nanoparticles of the outer surface is larger than the average size of the molecular sieve nanoparticles of the inner surface.

48. The preparation method according to any one of claims 33-47, **characterized in that** the molecular sieves are independently dispersed on the fiber surface.

49. The preparation method of claim 48, **characterized in that** the independent dispersion means that the molecular sieve microparticles are dispersed on the fiber surface with a gap between each other.

50. The preparation method of claim 48, **characterized in that** the independent dispersion means the minimum distance between the molecular sieve microparticle and the nearest molecular sieve microparticle is greater than or equal to one half of the sum of the particle sizes of the two molecular sieve microparticles.

51. The preparation method of claim 33, **characterized in that** the content of the molecular sieve accounts for 0.05 to 80 wt% of the molecular sieve/fiber composite material; preferably, the content of the molecular sieve accounts for 1 to 50 wt% of the molecular sieve/fiber composite material; preferably, the content of the molecular sieve accounts for 5 to 35 wt% of the molecular sieve/fiber composite material; preferably, the content of the molecular sieve accounts for 10 to 25 wt% of the molecular sieve/fiber composite material; more preferably, the content of the molecular sieve accounts for 15 to 20 wt% of the molecular sieve/fiber composite material.

52. The preparation method according to any one of claims 33-51, **characterized in that** the molecular sieve may be selected from the group consisting of aluminosilicate molecular sieve, phosphate molecular sieve, borate molecular sieve, heteroatom molecular sieve, and combination thereof.

53. The preparation method of claim 52, **characterized in that** the aluminosilicate molecular sieve is selected from the group consisting of X-type molecular sieve, Y-type molecular sieve, A-type molecular sieve, ZSM-5 molecular sieve, chabazite, β-molecular sieve, mordenite, L-type molecular sieve, P-type molecular sieve, merlinoite, and combination thereof.

54. The preparation method of claim 52, **characterized in that** the phosphate molecular sieve is selected from the group consisting of AlPO₄-5 molecular sieve, AlPO₄-11 molecular sieve, SAPO-31 molecular sieve, SAPO-34 molecular sieve, SAPO-11, and combination thereof.

55. The preparation method of claim 52, **characterized in that** the borate molecular sieve is selected from the group consisting of BAC-1 molecular sieve, BAC-3 molecular sieve, BAC-10 molecular sieve, and combination thereof.

56. The preparation method of claim 52, **characterized in that** the heteroatom molecular sieve is that a heteroatom element replaces part of silicon, aluminum, and phosphorus of the molecular sieve framework to form a molecular sieve containing other elements.

57. The preparation method of claim 56, **characterized in that** the heteroatom element is selected from the group consisting of transition metal elements in the fourth, fifth, sixth periods, and combination thereof.

58. The preparation method according to any one of claims 33-57, **characterized in that** the fiber is a polymer containing hydroxyl groups in a repeating unit.

59. The preparation method according to any one of claims 33-57, **characterized in that** the fiber is selected from the group consisting of silk fiber, chitin fiber, rayon fiber, acetate fiber, carboxymethyl cellulose, bamboo fiber, cotton fiber, linen fiber, wool, wood fiber, lactide polymer fiber, glycolide polymer fiber, polyester fiber, polyamide fiber, polypropylene fiber, polyethylene fiber, polyvinyl chloride fiber, polyacrylonitrile fiber, viscose fiber, and combination thereof.

60. A modified molecular sieve/fiber composite material, the modified material comprises an additive, and the molecular sieve/fiber composite material according to any one of claims 1-32; or the molecular sieve/fiber composite material prepared by the preparation method according to any one of claims 33-59.

61. The modified material of claim 60, **characterized in that** the additive is selected from the group consisting of metal, metal ion-containing compound, synthetic polymer compound, poorly soluble polysaccharide, protein, nucleic acid, pigment, antioxidant, mold inhibitor, detergent, surfactant, antibiotic, antibacterial agent, antimicrobial agent, anti-inflammatory agent, analgesic agent, antihistamine, and combination thereof.

62. The modified material of claim 61, **characterized in that** the synthetic polymer compounds are selected from the group consisting of polymer compounds prepared by self-addition polymerization reaction, polymer compounds obtained by polycondensation reaction, polyamide polymer compounds, synthetic rubber polymer compounds, and combination thereof.

63. The modified material of claim 62, **characterized in that** the proteins are selected from the group consisting of fibrin, collagen, oxidoreductase, transferase, hydrolase, lyase, isomerase, synthetase, and combination thereof.

64. The modified material of claim 63, **characterized in that** the transferase is selected from the group consisting of methyltransferase, aminotransferase, acetyltransferase, transferase, kinase, polymerase, and combination thereof.

65. The modified material of claim 63, **characterized in that** the hydrolase is selected from the group consisting of amylase, protease, lipase, phosphatase, glycosidase, and combination thereof.

66. The modified material of claim 63, **characterized in that** the lyase is selected from the group consisting of dehydratase, decarboxylase, carbonic anhydrase, aldolase, citrate synthase, and combination thereof.

67. The modified material of claim 63, **characterized in that** the synthetase is selected from the group consisting of glutamine synthetase, DNA ligase, biotin-dependent carboxylase, and combination thereof.

68. The modified material of claim 61, **characterized in that** the antibiotic is selected from the group consisting of quinolone antibiotic, β-lactam antibiotic, macrolide antibiotic, aminoglycoside antibiotic, and combination thereof.

69. The modified material of claim 61, **characterized in that** the poorly soluble polysaccharides are selected from the group consisting of natural non-chemically treated polymer polysaccharides, chemically modified polymer polysaccharides, and combination thereof.

70. The modified material of claim 69, **characterized in that** the modified polymer polysaccharides are selected from the group consisting of carboxy-modified polymer polysaccharides, amino-modified polymer polysaccharides, thiol-modified polymer polysaccharides and combination thereof.

71. The modified material of claim 69 or 70, **characterized in that** the polymer polysaccharides are selected from the group consisting of cellulose, lignin, starch, chitosan, agarose, and combination thereof.

72. A compound, **characterized in that** the compound comprises a molecular sieve/fiber composite material according to any one of claims 1-32 or a molecular sieve/fiber composite material prepared by the preparation method according to any one of claims 33-59.

73. The compound of claim 72, **characterized in that** the compound is a hemostatic material.

74. The compound of claim 73, **characterized in that** the hemostatic material is a hemostatic textile.

75. The compound of claim 74, **characterized in that** the hemostatic textile is selected from the group consisting of a hemostatic bandage, a hemostatic gauze, a hemostatic cloth, a hemostatic clothing, a hemostatic cotton, a hemostatic suture, a hemostatic paper, a hemostatic band-aid, and combination thereof.

76. The compound of claim 75, **characterized in that** the hemostatic clothing is a material worn on the human body for protection or decoration.

77. The compound according to claim 75 or 76, **characterized in that** the hemostatic clothing is selected from the group consisting of hemostatic underwear, hemostatic vest, hemostatic cap, hemostatic pants, and combination thereof.

78. The compound of claim 72, **characterized in that** the compound is an anti-radiation material.

79. The compound of claim 78, **characterized in that** the anti-radiation material is selected from the group consisting of anti-radiation clothing, anti-radiation umbrella, anti-radiation cap, and combination thereof.

80. The compound of claim 72, **characterized in that** the compound is an antibacterial material.

81. The application of the molecular sieve/fiber composite modified material according to any one of claims 60-71 in the fields of hemostasis, beauty, deodorization, sterilization, water purification, air purification, and radiation resistance.

82. The application of the compound according to any one of claims 72-80 in the fields of hemostasis, beauty, deodorization, sterilization, water purification, air purification, and radiation resistance.
